# EUROPÄISCHE PATENTSCHRIFT

(11) **EP 0 659 177 B1**
(45) Veröffentlichungstag und Bekanntmachung des Hinweises auf die Patenterteilung: **08.12.1999**
(21) Anmeldenummer: 93919299.3
(22) Anmeldetag: 08.09.1993
(51) Int. Cl.: C07D 205/095, C07D 413/12, C07D 413/14, C07D 403/12, A61K 31/395

(54) **4-OXO-AZETIDIN-2-SULFONSÄUREAMIDE UND IHRE SALZE, VERFAHREN ZU DEREN HERSTELLUNG UND IHRE VERWENDUNG**
4-OXO-AZETIDINE-2-SULPHONIC ACID AMIDES AND THEIR SALTS, PROCESS FOR THEIR PRODUCTION AND THEIR USE
AMIDES DE L'ACIDE 4-OXO-AZETIDINE-2-SULFONIQUE ET LEURS SELS, LEUR PROCEDE DE FABRICATION ET LEUR UTILISATION

(30) Priorität: 08.09.1992 DE 4230053
(43) Veröffentlichungstag der Anmeldung: 28.06.1995
(73) Patentinhaber: PLIVA Handels GmbH, 22083 Hamburg (DE); PLIVA FARMACEUTSKA KEMIJSKA PREHRAMBENA I KOZMETICKA INDUSTRIJA DIONICKO DRUSTVO ZAGREB, 41000 Zagreb (HR)
(72) Erfinder: KOVACEVIC, Mice, 41000 Zagreb (HR); HERAK, Jure, J., 41000 Zagreb (HR); MANDIC, Zora, 41000 Zagreb (HR); LUKIC, Irena, 41000 Zagreb (HR); TOMIC, Mirjana, 41000 Zagreb (HR); BRKIC, Zinka, 41000 Zagreb (HR)
(74) Vertreter: Finck, Dieter, Dr.Ing.
(86) Internationale Anmeldenummer: EP9302428
(87) Internationale Veröffentlichungsnummer: WO9405632

(56) Entgegenhaltungen:
- EP-A- 0 095 764
- EP-A- 0 109 816
- EP-A- 0 499 232
- DE-A- 2 556 045

## Beschreibung

Die vorliegende Erfindung betrifft neue 4-Oxo-azetidin-2-sulfonsäureamide und ihre Salze, Verfahren zu deren Herstellung und ihre Verwendung zur Herstellung von Arzneimitteln.

Es wurden einige 4-Oxo-Azetidin-sulfonsäuren, am bekanntesten die 4-Oxo-azetidin-1-sulfonsäuren beschrieben [Chemistry in Britain (1983) 302]; dazu wird das beta-Lactamantibiotik Aztreonam gezählt [Drugs of the Future **8** (1983) 295].

Es sind auch einige durch den Abbau von bizyklischen Molekeln [Angew. Chem. 95 (1983) 912], oder durch die Oxidation von entsprechenden 4-Oxo-Azetidin-2-sulfinsäurederivaten [YU Patentanmeldung P-250/91; YU Patentanmeldung P-1390/91 und EP-A-0 499 232] erhaltenen 4-Oxo-azetidin-2-sulfonsäuren bekannt.

Ebenso sind einige 4-Oxo-Azetidin-2-sulfonsäurederivate, wie z.B. Säurechloride [DE Patentanmeldung 2556071/76], -ester [Croat. Chem. Acta **62** (1989) 521-527] und -thioester [J. Chem. Soc. Chem. Commun. **23** (1972) 1304-1305], oder 1-sulfo-2-oxoazetidinderivate [EP-A-0 095 764] bekannt.

Gemäß den eigenen Kenntnissen der Anmelderin betreffend den Stand der Technik sind die 4-Oxo-azetidin-2-sulfonsäureamide und ihre Salze noch nicht bekannt.

Der Gegenstand der vorliegenden Erfindung sind neue 4-Oxoazetidin-2-sulfonsäureamide und ihre Salze der allgemeinen Formel I 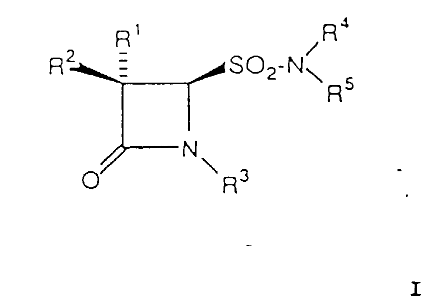 in welcher die Reste die folgende Bedeutung haben:
R¹ Wasserstoff, Halogen;
R² Wasserstoff, Halogen, NH₂, Phenyl-CH₂CONH, PhenylOCH₂CONH, Phthalimido o-MeNHCOC₆H₄CONH, Isoxazolylcarbonylamino;
R³ Wasserstoff, Me₂C=C-COOMe, H₂C=C(Me)-CH-COOMe, Me₂C=C-COOCH₂Phenyl, H₂C=C(Me)-CH-COOCH₂C₆H₄NO₂-p, Me₂C=C-COOCH₂C₆H₄NO₂-p, Me₂C=C-COOCH₂C₆H₄Me-m, H₂C=C(Me)-CHCO-OCH₂C₆H₄Me-m, Me₂C=C-COOH;
R⁴ Wasserstoff oder Natrium und
R⁵ Alkyl, Benzyl, Isoxazol oder Pyrazol.

Ein weiterer Gegenstand der vorliegenden Erfindung ist ein Verfahren zur Herstellung der neuen 4-Oxo-azetidin-2-sulfonsäureamide der allgemeinen Formel I, in welcher die zitierten Reste die obige Bedeutung haben, mittels Oxidierung von 4-Oxo-azetidin-2-sulfinsäureamiden der allgemeinen Formel II 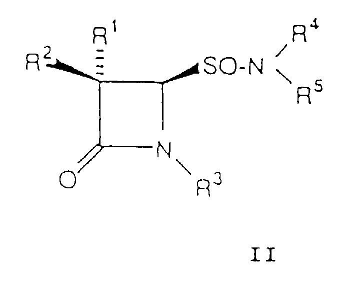 in welcher die Reste die folgenden Bedeutungen haben:
R¹ Wasserstoff oder Halogen;
R² Wasserstoff, Halogen, PhenylCH₂CONH, PhenylOCH₂CONH, Phthalimido, o-MeNHCOC₆H₄CONH, Isoxazolylcarbonylamino;
R³ Me₂C=C-COOMe, H₂C=C(Me)-CH-COOMe, Me₂C=C-COOCH₂Phenyl, H₂C=C(Me)-CH-COOCH₂C₆H₄NO₂-p, Me₂C=C-COOCH₂C₆H₄NO₂-p, Me₂C=C-COOCH₂C₆H₄Me-m, H₂C=C(Me)-CHCOOCH₂C₆H₄Me-m;
R⁴ Wasserstoff und
R⁵ Alkyl, Benzyl, Isoxazol oder Pyrazol.

Die Oxidation wird in den für die in der organischen Chemie bekannten Oxidationen üblichen Mitteln, wie z.B. Wasserstoffperoxid, Peracetessigsäure, m-Chlorperbenzoesäure und Kaliumpermanganat, in einem sauren oder neutralen, wäßrigen oder wäßrig-organischen Medium und bei einer Temperatur von 0 bis 100°C ausgeführt. Alle Reaktionen werden unter üblichen Reaktionsbedingungen und Molarverhältnissen und mittels standardisierten Isolierungsschritten ausgeführt. In Abhängigkeit von der Art des Oxidationsmittels, der Molarverhältnisse und der Reaktionstemperatur werden verschiedene Derivate der 4-Oxo-azetidin-2-sulfonsäureamide, wie in den einzelnen Beispielen angeführt ist, erhalten. In Abhängigkeit von der Bearbeitungsart und der Isolierung sind die 4-Oxo-azetidin-2-sulfonsäureamide in der Form von freien Amiden oder ihrer anorganischen Salze erhältlich. Die Amino- und Carboxyschutzgruppen werden gemäß üblichen bekannten Methoden abgespalten.

Die Sulfonamide der allgemeinen Formel II werden durch die Reaktion der entsprechenden Sulfinylchloride mit Aminen, wie im US Patent Nr. 4 052 387 (1977) beschrieben ist, hergestellt. Die Sulfinylchloride sind ausgehend von Penicillinsulfoxiden, wie im US Patent Nr. 4 081 440 (1978), oder aus Sulfinsäure, wie in Croat. Chem. Acta **62** (1989) 521 beschrieben wurde, in situ erhältlich. Ein weiterer Gegenstand der vorliegenden Erfindung ist die Verwendung der erfindungsgemäßen neuen Verbindungen als Zwischenverbindungen bei der Herstellung von verschiedenen beta-Lactamanalogen, insbesondere von neuen bizyklischen Systemen.

Ein weiterer Gegenstand der vorliegenden Erfindung ist die Verwendung der erfindungsgemäßen Verbindungen als Wirkstoffen bei der Herstellung von Präparaten mit antimikrobieller Wirksamkeit.

Die Erfindung ist durch die folgenden Beispiele illustriert.

### BEISPIEL 1

### (2R,3R) l-(1'-Methyloxycarbonyl-2'-methyl-prop-1'-enyl)-2-benzylaminosulfonyl-3-phenoxyacetamido-4-oxo-azetidin

Die Lösung des (5R,6R) 6-Phenoxyacetamidopenicillanat-sulfoxid-methylesters (1.9 g, 5 mMol) in wasserfreiem Toluol (215 ml) wird mit Kalziumoxid (1.65 g, 28.4 mMol) und N-Chlorsukzinimid (0.85 g, 6.4 mMol) versetzt und das Reaktionsgemisch im Stickstoffstrom bei Siedetemperatur 1.5 Stunden gerührt. Der Inhalt wird auf 0°C gekühlt, mit Benzylamin (2.26 g, 21 mMol) versetzt und 2 Stunden weitergerührt. Das Reaktionsgemisch wird filtriert, die Mutterlauge mit Wasser (2 x 90 ml) gewaschen, getrocknet (Na₂SO₄), filtriert und im Vakuum eingeengt. Der Rückstand wird auf einer Silikagelsäule mit einem Methylenklorid:Methanol-Lösungsmittelgemisch (20 : 1) eluiert. Das erhaltene Gemisch (1.8 g, 74.4%) der epimeren (2R,3R) 1-(1'-Methyloxycarbonyl-2'-methyl-prop-1'-enyl)-2-benzyl-aminosulfinyl-3-phenoxyacetoamido-4-xoazetidine [Isomer im Überschuß ¹H NMR (CDCl₃) 6: 2.13 und 2.26 (6H 2s, CMe₂), 3.75 (3H, s, OMe), 4.13-4.50 (5H, m, CH₂N, CH₂O, SNH), 4.95 (1H, d, J 5.0 Hz, C₂H), 5.87 (1H, dd, J 5.0 und 10.0 Hz, C₃H), 6.75-7.37 (5H, m, C₆H₅O), 8.47 (1H, d, J 10.0 Hz CONH) ppm] wird in Methylenchlorid (35 ml) und Ameisensäure (7 ml) gelöst, mit wäßriger (30%) H₂O₂-Lösung (28 ml) versetzt und das Reaktionsgemisch 6 Stunden bei Raumtemperatur gerührt. Der organische Extrakt wird abgetrennt, mit Wasser gewaschen, getrocknet (Na₂SO₄), filtriert und eingeengt. Es verbleibt ein schaumartiges Produkt (1.98 g, 95 %): R_{f} 0.55 (CH₂Cl₂:MeOH = 20:1);
IR (KBr) 3420-3230m, 1785s, 1735m, 1690s, 1605m, 1530s, 1495m, 1440m, 1370m, 1335m, 1225s, 1162s, 1065s, 995w cm⁻¹;
¹H NMR (CDCl₃) δ: 2.09 und 2.25 (6H, 2s, CMe₂), 3.69 (3H, s, OMe), 4.09-4.41 (5H, m, CH₂N, CH₂O, SNH), 4.80 (1H, d, J 5.3 Hz, C₂H), 5.83 (1H, dd, J und 10.8 Hz, C₃H), 6.87-7.44 (5H, m, C₆H₅O), 7.77 (1H, d, J 10.8 Hz, CONH) ppm.

### BEISPIEL 2

### (2R,3R) 1-(1'-Methyloxycarbonyl-2'-methyl-prop-2'-enyl)-2-[(5'-methyl-isoxazol-3'-yl)aminosulfonyl]-3-phenoxyacetamido-4-oxo-azetidin

Der (5R,6R) 6-Phenoxyacetamidopenicillanat-sulfoxid-methylester (1.9 g, 5 mMol) wird der Reaktion mit N-Chlorsukzinimid wie im Beispiel 1 angeführt, unterworfen, wonach an Stelle von Benzylamin mit 3-Amino-5-methylisoxazol (2.06 g, 21 mMol) versetzt wird. Nach dem Rühren und der Bearbeitung des Reaktionsgemisches wird das epimere Gemisch der (2R,3R) 1-(1'-Methyloxycarbonyl-2'-methyl-prop-2'-enyl)-2-[(5'-methyl-isoxazol-3'-yl)-aminosulfinyl]-3-phenoxyacetamido-4-oxo-azetidine isoliert. Nach Einengen der Toluollösung kristallisiert das Epimer mit einem Schmp.: 185-190°C [¹H NMR (CDCl₃) δ: 1.99 (3H, s, Me), 2.15 (3H, s, Me-Isoxazol), 3.85 (3H, s, OMe), 4.43 (2H, bs, OCH₂), 5.05 (1H, s, NCHCO), 5.09 und 5.27 (2H, 2bs, =CH₂), 5.37 (1H, d, J 4.5 Hz, C₂H), 5.79 (1H, s, CH-Isoxazol), 5.84 (1H, dd, J 4.5 und 9.5 Hz, C₃H), 6.95-7.34 (5H, m, C₆H₅O), 7.70 (1H, d, J 9.5 Hz, CONH) 8.29 (1H, s, SNH) ppm; Anal. C₂₁H₂₃O₈N₄S; gef.: C, 52.42; H, 5.82; N, 12.02; S, 6.34%; ber.: C, 52.93; H, 5.08; N, 11.76; S, 6.73%]. Die abgesaugten Kristalle werden in Methylenchlorid und Ameisensäure gelöst und die Oxidation mit Wasserstoffperoxid, wie im Beispiel 1 erwähnt, ausgeführt, wobei ein schaumartiges Produkt erhalten wird: R_{f} 0.20 (CH₂Cl₂:MeOH =20:1);
IR (KBr) 3400-3000m, 1795vs, 1750s, 1690m, 1620m, 1605m, 1535-1494s, 1465m, 1440m, 1245s, 1165s, cm⁻¹;
¹H NMR (CDCl₃) δ: 1.91 (3H, s, Me), 2.18 (3H, s, Me-Isoxazol), 3.79 (3H, s, OMe), 4.44 (2H, bs, OCH₂), 4.90 (1H, s, NCHCO), 5.00 und 5.17 (2H, 2bs, =CH₂), 5.46 (1H, d, J 5.0 Hz, C₂H), 6.01 (1H, dd, J 5.0 und 10.3 Hz, C₃H), 6.82-7.41 (5H, m, C₆H₅O), 7.75 (1H, d, J 10.3 Hz, CONH) ppm.

### BEISPIEL 3

### (2R,3R) 1(1'-Methyloxycarbonyl-2'-methyl-prop-1'-enyl)-2-[(5'-methyl-isoxazol-3'-yl)aminosulfonyl]-3-phenoxacetamido-4-oxo-azetidin

Das gemäß Beispiel 2 hergestellte Sulfinamid (Schmp. .185-190°C), wird in Methylenchlorid gelöst und mit Triethylamin 2 Stunden bei Raumtemperatur gerührt. Nach Bearbeitung des Reaktionsgemisches mittels Chromatographie an einer Silikagelsäule wird das (2R,3R) 1-(1'-Methyloxycarbonyl-2'-methylprop-1'-enyl)-2-[5'-methyl-isoxazol-3'-yl)-aminosulfinyl]-3-phenoxyacetamido-4-oxo-azetidin in einer 80%-iger Ausbeute erhalten: ¹H NMR (CDCl₃) δ: 2.06 und 2.19 (6H, 2s, CMe₂), 2.25 (3H, s, Me-Isoxazol), 3.76 (3H, s, OMe), 4.31 und 4.40 (2H, ABq, J 15.0 Hz, OCH₂), 5.34(1H, d, J 5.0 Hz, C₂H), 5.80 (1H, s, CH-Isoxazol), 5.60 (1H, dd, J 5.0 und 8.8 Hz, C₃H), 6.84-7.32 (5H, m, C₆H₅O=, 7.92 (1H, d, J 8.8 Hz, CONH), 8.43 (1H, s, SNH= ppm], welches anschließend mit Wasserstoffperoxid wie im Beispiel 1 angeführt, oxidiert wird.
Es wird ein schaumartiges Produkt in einer 85%-iger Ausbeute erhalten. R_{f} 0.24 (CH₂Cl₂:MeOH=20:1);
IR (KBr) 1790s, 1735m, 1700s, 1620m, 1540-1495s, 1465m, 1440m, 1380m, 1230s, 1165s cm⁻¹;
¹H NMR (CDCl₃) 6: 2.09 und 2.22 (6H 2s, CMe₂), 2.26 (3H, s, Me-Isoxazol), 3.71 (3H, s, OMe), 4.46 und 4.57 (2H, ABq J 15.0 Hz, OCH₂), 4.57 (1H, s, SNH), 5.54 (1H, d, J 5.2 Hz, C₂H), 6.07 (1H, s, CH-Isoxazol), 6.06 (1H, dd, J 5.2 und 10.4 Hz, C₃H), 6.95-7.37 (5H, m, C₆H₅O), 7.76 (1H, d, J 10.4 Hz, CONH), ppm.

### BEISPIEL 4

### (2R,3R) 1-(1'-p-Nitrobenzyloxycarbonyl-2'-methyl-prop-2'-enyl)-2-[(5'-methyl-isoxazol-3'-yl)aminosulfonyl]-3-phthalimido-4-oxo-azetidin

Der (5R,6R) 6-Phthalimidopenicillanat-sulphoxid-p-nitrobenzylester (1.5 g, 3 mMol) wird der Reaktion mit N-Chlorsukzinimid (0.4 g, 3 mMol), wie im Beispiel 1 angeführt, unterworfen, wonach mit 3-Amino-5-methyl-isoxazol (1.18 g, 12 mMol) versetzt und das Reaktionsgemisch 4 Stunden bei 10°C gerührt wird. Die Toluollösung wird dekantiert, mit Wasser gewaschen, getrocknet, filtriert und im Vakuum eingeengt. Mittels Chromatographie auf einer Silikagelsäule in einem Methylenchlorid:Ethylacetat-Lösungsmittelsystem (4:1) werden 0.88 g (2R,3R) 1-(1'-Nitrobenzyloxycarbonyl-2'-methyl-prop-2'-enyl)-2-[5'-methyl-isoxazol-3'-yl)aminosulfinyl]-3-phthalimido-4-oxo-azetidin erhalten [¹H NMR (CDCl₃) δ: 1.90 (3H, s, Me), 2.27 (3H, s, Me-Isoxazol), 4.76 (1H, bs, NCHCO), 4.92 und 5.06 (2H, 2bs, =CH₂), 5.25 (2H, bs, OCH₂), 5.67 (1H, d, J 5.4 Hz, C₂H), 6.09 (1H, d, J 5.4, Hz, C₃H), 7.20 (1H, bs, CH-Isoxazol), 7.52 und 8.20 (4H, 2d, J 9.04 Hz, C₆H₄NO₂), 7.71-7.95 (4H, m, Phthalimido), und 8.05 (1H, bs, SNH) ppm]. Die Reaktion mit Wasserstoffperoxid (12 ml, 30%-ige wäßrige Lösung) in Methylenchlorid (15 ml) und Ameisensäure (2 ml), wie im Beispiel 1 angeführt, ergibt 0.63 g Sulfonamid:
R_{f} 0.58 (CH₂Cl₂:MeOH-9:1);
IR (KBr) 1805s, 1790s, 1735vs, 1615m, 1525m, 1475m, 1390s, 1350s, 1270w, 1170w, 1110w, 720m cm⁻¹;
¹H NMR (CDCl₃) δ: 2.02 (3H, s, Me), 2.22 (3H, s, Me-Isoxazol), 4.98 (1H, s, NCHCO), 5.09-5.35 (4H, m, =CH₂, OCH₂), 5.57 (1H, d, J 4.5 Hz, C₂H), 5.76 (1H, d, J 4.5 Hz, C₃H), 5.97 (¹H, s, CH-Isoxazol), 7.51 und 8.15 (4H, 2d, J 8.4 Hz, C₆H₄NO₂), 7.69-8.05 (4H, m, Phthalimido) ppm].

### BEISPIEL 5

### (2R,3R) 2-Benzylaminosulfonyl-3-phenylacetamido-4-oxo-azetidin

Der (5R,6R) 6-Phenylacetamidopenicillanat-sulfoxid-p-nitrobenzylester (2.10 g, 4.3 mMol) wird der Reaktion mit N-Chlorsukzinimid (0.72 g, 5.4 mMol), wie im Beispiel 1 beschrieben, unterworfen und anschließend wird mit Benzylamin (1.5 ml) das epimere Gemisch der (2R,3R) 1-(1'-p-Nitrobenzyloxycarbonyl-2'-methyl-prop-1'-enyl)-2-benzyl-amino-sulfinyl-3-phenylacetamido-4-oxo-azetidine (1.78 g, 69.4%) hergestellt; [Epimer im Überschuß ¹H NMR (DMSO-d₆) δ: 2.11 und 2.25 (6H, 2s, CMe₃), 3.53 (2H, s, CH₂CO), 3.81-4.36 (3H, m, SNHCH₂), 4.78 (1H, d, J 5.4 Hz, C₂H), 5.24 (2H, bs, OCH₂), 5.70 (1H, d, J 5.4 und 9.0 Hz, C₃H), 6.65 (1H, d, J 9.0 Hz, CONH), 7.07-7.35 (10H, m, 2C₆H₅), 7.44 und 8.17 (4H, 2d, J 9.0 Hz, C₆H₄NO₂) ppm]. Das Produkt wird in Ethylacetat (25 ml) und 80%-iger Essigsäure (25 ml) gelöst und eine wäßrige 4%-ige Kaliumpermanganatlösung (50 ml) im Laufe von 1 Stunde bei einer Temperatur von 0°C zugetropft. Es wird eine 30%-ige Wasserstoffperoxidlösung bis zum Verschwinden der Permanganatfarbe zugetropft. Der organische Extrakt wird abgetrennt, mit einer wäßrigen NaHCO₃-Lösung und Wasser gewaschen, getrocknet (Na₂SO₄) und im Vakuum eingeengt. Mittels Chromatographie an einer Silikagelsäule und Eluierung in einem Methylenchlorid : Ethylacetat - Lösungsmittelsystem (4:1) werden 0.3 g (23.5%) des Sulfonamids mit einem Schmp. 135-137°C abgeschieden:
R_{f} 0.92 (n-BuOH:HOAc:H₂O=4:1:1);
IR (KBr) 3360m, 3290m, 1770vs, 1655s, 1515m, 1320s, 1135s, 720s cm⁻¹;
¹H NMR (DMSO-d₆) δ: 3.56 (2H, bs, CH₂CO), 4.15-4.20 (2H, m, NCH₂, 4.85 (1H, d, J 4.7 Hz, C₂H), 5.57 (1H, dd, J 4.7 und 9.5 Hz, C₃H), 7.25 und 7.38 (10H, 2 bs, 2C₆H₅), 7.87-7.93 (1H, m, SNH), 8.49 (1H, d, J 9.5 Hz, CONH), 9.24 (1H, bs, N₁H) ppm;
Anal. C₁₈H₁₉N₃O₄S
Gef.: C 57.70, H 5.90, N 11.74, S 8.47%
Ber.: C 57.89, H 5.13, N 11.25, S 8.59%.

### BEISPIEL 6

### (2R,3R) 1-(1'-Carboxyl-2'-methyl-prop-1'-enyl)-2-[(5'-methyl-isoxazol-3'-yl)amino-sulfonyl]-3-phenylacetamido-4-oxo-azetidin

Der (5R,6R) 6-Phenylacetamidopenicillanat-sulfoxid-p-nitrobenzylester (3.0 g, 6.2 mMol) wird der Reaktion mit N-Chlorsukzinimid (1.0 g, 7.5 mMol) wie im Beispiel 1 angeführt, unterworfen, wonach mit 3-Amino-5-methyl-isoxazol (2.4 g, 25 mMol) versetzt und das Reaktionsgemisch 3 Stunden 5°C gerührt wird. Die Toluollösung wird dekantiert, mit Wasser gewaschen, getrocknet und im Vakuum eingeengt. Es werden 1.43 g (2R,3R) 1-(1'-p-Nitrobenzyl-oxycarbonyl-2'-methylprop-2'-enyl)-2-[(5'-methyl-isoxazol-3'-yl)-amino-sulfinyl]-3-phenylacet-amido-4-oxo-azetidin erhalten [Schmp. 157-160°C; ¹H NMR (CDCl₃) δ: 1.93 (3H, s, Me), 2.35 (3H, s, Me-Isoxazol), 3.61 (2H, s, CH₂CO), 4.94 (1H, bs, NCHCO), 5.07 und 5.19 (2H, 2bs, =CH₂), 5.13 (1H, d, J 4.8 Hz, C₂H), 5.28 (2H, bs, OCH₂), 5.57 (1H, bs, CH-Isoxazol), 5.77 (1H, dd, J 4.8 und 9.0 Hz, C₃H), 7.22 (5H, bs, C₆H₅), 7.44 (1H, d, J 9.0 Hz, CONH), 7.49 und 8.21 (4H, 2d, J 8.8 Hz, C₆H₄NO₂) ppm], welches unter Rühren in Triethylamin und Methylenchlorid in das (2R,3R) 1-(1'-p-Nitrobenzyloxycarbonyl-2'-methyl-prop-1'-enyl)-2-[(5'-methyl-Isoxazol-3'-yl)aminosulfinyl]-3-phenylacetamido-4-oxo-azetidin übergeht [¹H NMR (CDCl₃) δ: 2.18 und 2.22 (6H, 2s, CMe₂), 2.34 (3H, s, Me-Isoxazol), 3.61 (2H, s, CH₂CO), 5.11 (1H, d, J 4.9 Hz, C₂H), 5.25 (2H, s, OCH₂), 5.64 (1H, dd, J 5.0 und 8.4 Hz, C₃H), 5.63 (1H, s, CH-Isoxazol), 7.24 (5H, s, C₆H₅), 7.26 (1H, d, J 8.4 Hz, CONH), 7.47 und 8.18 (4H, 2d, J 8.5 Hz, C₆H₄NO₂) ppm]. Das erhaltene Sulfinamid wird mit Wasserstoffperoxid, wie im Beispiel 1 angeführt, in das (2R,3R) 1-(1'-p-Nitrobenzyloxycarbonyl-2'-methyl-prop-1'-enyl)-2-(5'-methyl-isoxazol-3'-yl )aminosulfonyl-3-phenylacetamido-4-oxo-azetidin oxidiert.

[R_{f} 0.57 (CH₂Cl₂:MeOH=8:1) IR (KBr) 3680-2500m, 1785s, 1730m, 1665s, 1615m, 1520s, 1350s, 1215m, 1160m cm⁻¹; ¹H NMR (CDCl₃) 6: 1.99 und 2.15 (6H, 2s, CMe₂), 2.28 (3H, s, Me-Isoxazol), 3.59 (2H, s, CH₂CO), 5.19 (2H, s, OCH₂), 5.44 (1H, d, J 5.3 Hz, C₂H), 5.81(1H, s, CH-Isoxazol), 5.84 (1H, dd, J 5.3 und 9.9 Hz, C₃H), 6.84 (1H, d, J 9.9 Hz, CONH), 7.28 (5H, s, C₆H₅), 7.45 und 8.17 (4H, 2d, J 8.9 Hz, C₆H₄NO₂) ppm]. Das erhaltene Sulfonamid (0.35 g, 0.6 mMol) wird anschließend in Methanol (25 ml) gelöst und 2 Stunden bei einem Druck von 2.4 bar mit 10% Palladium auf Kohle (50 mg) hydriert. Das Reaktionsgemisch wird filtriert und die Mutterlauge im Vakuum eingeengt. Der Rückstand wird in Methylenchlorid (20 ml) und Wasser (20 ml) gelöst und auf einen pH-Wert von 8.5 durch Zugabe von Natriumhydrogenkarbonat eingestellt. Der wäßrige Extrakt wird abgetrennt, mit frischem Methylenchlorid ausgeschütelt, eine neue Portion Methylenchlorid zugegeben (15 ml) und der pH-Wert auf 2.2 mittels Zugabe von Salzsäure eingestellt. Der organische Extrakt wird abgetrennt, getrocknet (Na₂SO₄), filtriert und im Vakuum eingeengt. Es werden 0.14 g (52.4%) des Produktes erhalten. R_{f} 0.48 (CH₂Cl₂:MeOH-3:2)
IR (KBr) 3660-2300bm, 2910m, 1790s, 1680bs, 1620s, 1465m, 1270m, 1165m, 930w cm⁻¹;
¹H NMR (CDCl₃) δ: 1.93 und 2.07 (6H, 2s, CMe₂), 2.35 (3H, s, Me-Isoxazol), 3.67 (2H, s, CH₂CO), 5.68 (1H, d, J 5.2 Hz, C₂H), 6.0 (1H, dd, J 5.2 und 9.3 Hz, C₃H), 6.12 (1H, s, CH-Isoxazol), 6.63 (1H, d, J 9.3 Hz, CONH), 7.27-7.31 (5H, m, C₆H₅) ppm.

### BEISPIEL 7

### (2R,3R) 1-(1'-m-Methylbenzyloxycarbonyl-2'-methyl-prop-2'-enyl)-2-[(5'-methyl-isoxazol-3'-yl)aminosulfonyl]-3-[(3'-o-chlorphenyl-5'-methyl-isoxazol-4'-yl)karboxamido]-4-oxo-azetidin

Der (5R,6R) 6-(3'-o-Chlorphenyl-5'-methyl-isoxazol-4'-yl)-carboxamido-penicillanat-sulfoxid-m-methylbenzylester (2.0 g, 3.6 mMol) wird der Reaktion mit N-Chlorsukzinimid wie im Beispiel 1 angeführt, unterworfen, wonach das 3-Amino-5-methyl-isoxazol (1.1 g, 11 mMol) zugegeben wird und das Reaktionsgemisch 3 Stunden bei Raumtemperatur gerührt wird. Das Gemisch wird filtriert, die Mutterlauge mit Wasser (3 x 60 ml) extrahiert, getrocknet (Na₂SO₄), filtriert und im Vakuum eingeengt. Es wird ein epimeres Gemisch (1.92 g) der (2R,3R) 1-(1'-m-Methyl-benzyloxycarbonyl-2'-methyl-prop-2'-enyl)-2-[(5'-methyl-isoxazol-3'-yl)aminosulfinyl]-3-[(3'-o-chlorphenyl-5'-methyl-isoxazol-4'-yl)karboxamido]-4-oxo-azetidine [R_{f} 0.40 und 0.26 in Methylenchlorid : Ethylacetat (4:1)] erhalten. Das Produkt wird in Methylenchlorid und Ameisensäure gelöst und der Oxidierung mit Waserstoffperoxid, wie im Beispiel 1 angeführt, unterworfen. Das Rohprodukt wird an einer Silikagelsäule und durch Eluierung mit einem Methylenchlorid : Ethylacetat - Lösungsmittelgemisch chromatographiert. Es werden 0.62 g (44%) des Sulfonamids erhalten:
R_{f} 0.55 (CH₂Cl₂:MeOH=10:1);
IR (KBr) 3405w, 1795vs, 1745s, 1680vs, 1620s, 1520s, 1465s, 1385m, 1340m, 1265m, 1160m, 770m cm⁻¹;
¹H NMR (CDCl₃) δ: 1.79 (3H, s, Me), 2.31 (3H, s, Phenyl-Me), 2.35 und 2.72 (6H, 2s, 2Me-Isoxazol) 4.85 (1H, s, NCHCO), 4.90 und 5.10 (2H, 2s, =CH₂), 5.08 und 5.13 (2H, ABq, J 12 0 Hz, OCH₂), 5.44 (1H, d, J 5.0 Hz, C₂H), 5.95 (1H, dd, J 5.0 und 9.6 Hz, C₃H), 6.04 (1H, s, CH-Isoxazol), 6.37 (1H, d, J 9.6 Hz, CONH), 7.01-7.26 und 7.41-7.55 (8H, 2m, 2C₆H₄) ppm.

### BEISPIEL 8

### (2R) l-(1'-Benzyloxycarbonyl-2'-methyl-prop-1'-enyl)-2-benzylaminosulfonyl-3,3-dibromo-4-oxo-azetidin

Der (5R) 6,6-Dibromopenicillanat-sulfoxid-benzylester (7.0 g, 15 mMol) wird der Reaktion mit N-Chlorsukzinimid, wie im Beispiel 1 angeführt, unterworfen, wonach die Reaktion mit Benzylamin (4 ml, 37.5 mMol) weitergeführt wird. Das Reaktionsgemisch wird abgesaugt, die Mutterlauge mit Wasser gewaschen, getrocknet (Na₂SO₄), filtriert und im Vakuum eingeengt. Das Rohprodukt wird an einer Silikagelsäule mittels Eluierung mit einem Methylenchlorid : Ethylacetat-Lösungsmittelgemisch (6:1) chromatographiert, wobei 3.08 g (36%) des (2R) 1-(1'-Benzyloxycarbonyl-2'-methyl-prop-1'-enyl)-2-benzylamino-sulfinyl-3,3-dibrom-4-oxo-azetidins [¹H NMR (CDCl₃) d: 1.84 und 2.26 (6H, 2s, CMe₂), 3.68-4.40 (3H, m, SNHCH₂), 5.07 und 5.24 (2H, ABq, J 12 Hz, OCH₂), 5.09 (1H, s, C₂H), 7.10-7.30 (10H, m, 2C₆H₅) ppm], welches in Chloroform mit m-Chlor-perbenzoesäure (1.2 g, 6 mMol) oxidiert wird, erhalten werden. Das Reaktionsgemisch wird 20 Minuten bei -10°C und anschließend 1 Stunde bei Raumtemperatur gerührt. Es wird mit 1M Natriumbisulfitlösung (36 ml, 6 mMol) versetzt, die organische Schicht wird abgetrennt, mit Wasser gewaschen, getrocknet (Na₂SO₄), filtriert und im Vakuum eingeengt.

Der Rückstand wird in Methylenchlorid gelöst und durch eine Silikagelsäule geführt, wobei 2.1 g (59%) eines weißen krystallinischen Produktes mit einem Schmp. 120-122°C erhalten werden:
Rf 0.88 (CH₂Cl₂/EtOAC-4:1);
IR (KBr): 3250vs, 1780vs, 1730vs, 1640s, 1444vs, 1370b, 1255s, 1200s, 1165vs, 1055vs, 755vs, 700vs cm⁻¹;
¹H NMR (CDCl₃) δ: 2.09 und 2.28 (6H, 2s, CMe₂), 4.09 (2H, d, J 5.8 Hz, NCH₂Phenyl), 4.63 (1H, t, J 5.8 Hz, SNH), 5.08 und 5.34 (2H, ABq, J 11.7 Hz, OCH₂), 5.32 (1H, s, C₂H), 7.29-7.35 (10H, m, 2C₆H₅), ppm.

### BEISPIEL 9

### (2R) 1-(1'-Benzyloxycarbonyl-2'-methyl-prop-1'-enyl)-2-[(5'-methyl-isoxazol-3'-yl)amino-sulfonyl]-3,3-dibromo-4-oxo-azetidin

### a)

Der (5R) 6,6-Dibrompenicillanat-sulfoxid-benzylester (7.0 g, 15 mMol) wird der Reaktion mit N-Chlorsukzinimid wie im Beispiel 1 angeführt, unterworfen, anschließend wird mit 3-Amino-5-methyl-isoxazol (4.47 g, 45 mMol) versetzt und das Reaktionsgemisch 3 Stunden bei einer Temperatur von 20°C gerührt. Der Niederschlag wird abgesaugt, getrocknet, in Methylenchlorid (30 ml) gelöst und mit Triethylamin (1.5 ml) 1 Stunde bei einer Temperatur von 20°C gerührt. Das Reaktionsgemisch wird mit 0.1 N Salzsäure (pH-Wert von 1-2) und Wasser gewaschen, der organische Extrakt wird getrocknet (Na₂SO₄), filtriert und im Vakuum eingeengt. Der Rückstand besteht aus 1.85 g (2R) 1-(1'-Benzyloxycarbonyl-2'-methyl-prop-1'-enyl)-2-[(5'-methyl-isoxazol-3'-yl)amino-sulfinyl]-3,3-dibromo-4-oxo-azetidin [Schmp. 58-60°C; R_{f} 0.51 (CH₂Cl₂ : EtOAc=4:1); ¹H NMR (CDCl₃) δ: 1.88 und 2.13 (6H, 2s, CMe₂), 2.31 (3H, s, Me-Isoxazol), 5.13 (2H, s, OCH₂), 5,56 (1H, s, C₂H), 5.67 (1H, s, CH-Isoxazol), 7.35 (5H, s, C₆H₅) und 8.32 (1H, s, SNH) ppm], welcher mit m-Chlorperbenzoesäure wie im Beispiel 8 beschrieben, oxidiert wird. Das Rohprodukt wird an einer Silikagelsäule mittels Eluierung mit einem Methylenchlorid : Ethylacetat-Lösungsmittelgemisch (4:1) chromatographiert, wobei ein weißes kristallinisches Produkt (52.6%), Schmp. 168-170°C, erhalten wird:
R_{f} 0.25 (CH₂Cl₂:EtOAC-4:1);
IR (KBr): 3160m, 1780vs, 1765vs, 1625s, 1500s, 1395vs, 1383s, 1220s, 1175vs cm⁻¹;
¹H NMR (CDCl₃) δ: 2.06 und 2.15 (6H, 2s, CMe₂), 2.37 (3H, s, Me-Isoxazol), 5.05 (2H, s, OCH₂), 5.70 (1H, s, C₂H), 6.07 (1H, s, CH-Isoxazol), 7.31 (5H, s, C₆H₅) ppm.

### b)

Das gemäß dem obigen Verfahren hergestellte Sulfinamid (0.85 g, 1.5 mMol) wird in Methylenchlorid (5 ml) und Ameisensäure (5 ml) gelöst und mit Wasserstoffperoxid (0.56 ml) im Laufe von 1 Stunde bei Siedetemperatur oxidiert. Das gekühlte Reaktionsgemisch wird mit Wasser versetzt, die organische Schicht abgetrennt, mit 5 %-iger NaHCO₃-Lösung und Wasser geschütelt, getrocknet (Na₂SO₄), filtriert und eingeengt. Es wird ein identisches Produkt, wie beim Verfahren a), erhalten.

### BEISPIEL 10

### (2R) 1-(1'-Carboxyl-2'-methyl-prop-1'-enyl)-2-benzylaminosulfonyl-3,3-dibromo-4-oxo-azetidin

Die eisgekühlte Suspension von Aluminiumtrichlorid (0,4 g, 3 mMol) in Methylenchlorid (15 ml) wird im Stickstoffstrom mit der Lösung von (2R) 1-(1'-Benzyloxycarbonyl-2'-methyl-prop-1'-enyl)-2-benzylaminosulfonyl-3,3-dibromo-4-oxo-azetidin (0.59 g, 1 mMol) und Anisol (0.65 g, 6 mMol) in Methylenchlorid (15 ml) versetzt und 30 Minuten bei Raumtemperatur gerührt. Die Reaktionslösung wird mit Ethylacetat (15 ml) und 0.1 N Chlorwasserstoffsäure (5 ml) versetzt. Die Schichten werden getrennt und die Ethylacetatschicht mit 5%-iger Natriumhydrogenkarbonatlösung (2 x 20 ml) extrahiert. Die Schichten werden erneut getrennt. Der wäßrige Extrakt wird auf einen pH-Wert von 1 mittels 0.1 N Chlorwasserstoffsäure eingestellt, mit frischem Ethylacetat (20 ml) und mit Natriumchlorid versetzt und gut ausgeschüttelt. Die Ethylacetatschicht wird abgetrennt, erneut mit einer gesätigten Salzlösung gewaschen, anschließend getrocknet und im Vakuum eingeengt. Man erhält das Produkt: 0.49 g (98%); Schmp. 47-50°C:
R_{f} 0.66 (EtOAc:MeOH=3:1);
IR (Film): 3300m, 2960-2930m, 1805vs, 1700s, 1625m, 1425m, 1350s, 1160s cm-1;
¹H NMR (DMSO-d₆) δ: 1.98 und 2.23 (6H, 2s, CMe₂), 4.09 und 4.20 (2H, ABX, J 5.7, 6.0 und 15.2 Hz, CH₂Ph), 5.51 (1H, s, C₂H), 7.29-7.39 (5H, m, C₆H₅), 8.49 (1H, dd, J 5.7 und 6.0 Hz, NH), 13.5 (1H, b, COOH) ppm.

### BEISPIEL 11

### (2R) 1-(1'-Carboxyl-2'-methyl-prop-1'-enyl)-2-benzylaminosulfonyl-4-oxo-azetidin

Die (2R) 1-(1'-Benzyloxycarbonyl-2'-methyl-prop-1'-enyl)-4-oxo-azetidin-2-sulfinsäure (3.23 g, 10 mMol) wird in Thionylchlorid (20 ml) gelöst und die Lösung 1 Stunde bei 25°C gerührt. Der Thionylchlorid-Überschuß wird unter vermindertem Druck bis zu einem öligen Rückstand eingeengt, in Methylenchlorid (50 ml) gelöst, die Lösung auf 10°C abgekühlt und unter Rühren und Kühlen mit 5%-iger Benzylaminlösung in Methylenchlorid bis zu einem pH-Wert von 7.0 versetzt und bei dieser Temperatur 30 Minuten weitergerührt. Das abgeschiedene Benzylaminhydrochloridsalz wird abgesaugt und mit Methylenchlorid gewaschen. Die Mutterlauge wird mit Wasser (50 ml) versetzt, das Gemisch mit 10%iger Salzsäure bis zu einem pH-Wert von 1.3 angesäuert und die Schichten gelöst, die organische Schicht erneut mit Wasser gewaschen, über Natriumsulfat getrocknet und zur Trockne eingedampft. Es wird ein Gemisch (3.85 g, 93%) von epimeren (2R) 1-(1'-Benzyloxycarbonyl-2'-methyl-prop-1'-enyl)-2-benzylominosulfinyl-4-oxo-azetidinen mit Rf-Werten von 0.64 und 0.72 (CH₂Cl₂:EtOAC-2:1) erhalten, welche mittels Chromatographie an einer Silikagelsäule getrennt werden. [Substanz mit Rf-Wert von 0.64 : IR (CH₂Cl₂): 3020vs, 2980s, 1760vs, 1700m, 1415m, 1260vs, 1210s, 1070m, 900s, cm⁻¹; ¹H NMR (CDCl₃) δ: 1.95 und 2.24 (6H, 2s, CMe₂), 3.11 (1H, dd, J 5.0 und 15.3 Hz, α C₃H), 3.39 (1H, dd, J 2.6 und 15.3 Hz, β C₃H), 4.0-4.3 (3H, m, NHCH₂), 4.77 (1H, 2d, J 2.6 und 5.0 Hz, C₂H), 5.08 und 5.29 (2H, ABq, J 12.0 Hz, CH₂Phenyl), 7.32 (10 H, s, 2C₆H₅) ppm; [Substanz mit Rf-Wert von 0.72: IR (CH₂Cl₂): 3025vs, 2980s, 1755vs, 1700m, 1420m, 1260vs, 1210s, 1075m, 900s cm⁻¹; ¹H NMR (CDCl₃) δ: 2.08 und 2.21 (6H, 2s, CMe₂), 2.94 und 3.16 (2H, 2d, J 3.2 und 4.7 Hz, β C₃H und α C₃H), 4.19 (3H, b, NHCH₂), 4.77 (1H, dd, J 3.2 und 4.7 Hz, C₂H), 5.06 und 5.28 (2H, ABq, J 12.3 Hz, CH₂Phenyl), 7.32 (10 H, s, 2C₆H₅) ppm]. Das Sulfinamid (1.65 g, 4 mMol) wird der Reaktion mit Kaliumpermanganat wie im Beispiel 5 angeführt, unterworfen. Nach kurzem Verbleiben der Kaliumpermanganatfarbe ist die Reaktion beendet. Es werden 1.32 g (77%) (2R) 1-(1'-Benzyloxycarbonyl-2'-methyl-prop-1'-enyl)-2-benzylaminosulfonyl-4-oxo-azetidin, welches durch Chromatographie an einer Silikagelsäule mittels Eluierung mit einem Lösungsmittelgemisch CH₂Cl₂ : EtOAc-2:1 gereinigt wird, erhalten. [R_{f} 0.62 (Benzol:EtOAc-2:1); Schmp.: 92-94°C; IR (KBr): 3300vs, 1775vs, 1650vs, 1430m, 1350s, 1335s, 1290vs, 1200m, 1150s, 1070w cm⁻¹; ¹H NMR (CDCl₃) δ: 2.05 und 2.23 (6H, 2s, CMe₂), 3.16 (2H, d, J 3.8 Hz, α C₃H und β C₃H), 4.08 (2H, d, J 5.9 Hz, NCH₂), 4.50 (1H, t, J 5.9 Hz, NH), 4.89 (1H, t, J 3.8 Hz, C₂H), 5.09 und 5,18 (2H, ABq, J 12.0 Hz, CH₂Phenyl), 7.25 und 7.34 (10H, 2s, 2C₆H₅)ppm].

Das erhaltene Sulfonamid (0.4 g, 1.16 mMol) wird wie im Beispiel 6 beschrieben, hydriert und bearbeitet, wobei 0.36 g der Säure erhalten werden:
Rf 0.88 (n-BuOH:HAc:H₂O-4:1:1);
IR (KBr): 3260s, 1760vs, 1700s, 1630m, 1430m, 1330s, 1170s, 1070m cm⁻¹;
¹H NMR (CDCl₃) δ: 2.09 und 2.26 (6H, 2s, CMe₂), 3.17 (2H, d, J 4.1 Hz, C₃H), 4.26 (2H, s, NCH₂), 4.98 (1H, t, J 4.1 Hz, C₂H), 7.30 (5H, s, C₆H₅) ppm.

### BEISPIEL 12

### (2R) 2-Benzylaminosulfonyl-4-oxo-azetidin

### a)

Die (2R) 1-(1'-Methyloxycarbonyl-2'-methyl-prop-1'-enyl)-4-oxo-azetidin-2-sulfinsäure (5.0 g, 20 mMol) wird in Thionylchlorid (20 ml) gelöst und bearbeitet, wie im Beispiel 11 beschrieben ist. Es wird ein Gemisch (5.4 g, 80.4%) der epimeren (2R) 1-(1'-Methyloxycarbonyl-2'-methyl-prop-1'-enyl)-2-benzylaminosulfinyl-4-oxo-azetidine mit R_{f}-Werten von 0.20 und 0.27 (CH₂Cl₂:EtOAC-2:1) erhalten, welche mittels Chromatographie an einer Silikagelsäule getrennt werden [Substanz mit R_{f}-Wert 0.20 : IR (CH₂Cl₂): 3200-3300m, 2930m, 1760vs, 1715s, 1220s, 1080s cm⁻¹; ¹H NMR (CDCl₃) δ: 1.99 und 2.23 (6H, 2s, CMe₂), 3.17 (1H, dd, J 5.0 und 15.2 Hz, α C₃H), 3.46 (1H, dd, J 2.6 und 15.2 Hz, β C₃H), 3.75 (3H, s, OCH₃), 4.15-4.30 (1H, b, NH), 4.23 (2H, s, CH₂Phenyl), 4.92 (1H, 2d, C₂H J 2.6 und 5.0 Hz), 7.31 (5H, s, C₆H₅) ppm; Substanz mit Rf-Wert 0.27: IR (CH₂Cl₂): 3300m, 2950m, 1775vs, 1720s, 1360s, 1220s, 1080s cm⁻¹; ¹H NMR (CDCl₃) δ: 2.07 und 2.20 (6H, 2s, CMe₂), 2.97 (1H, dd, J 3.1 und 15.5 Hz, β C₃H), 3.25 (1H, dd, J 5.0 und 15.5 Hz, α C₃H), 3.73 (3H, s, OCH₃), 4.15-4.33 (1H, b, NH), 4.26 (2H, s, CH₂Ph), 4.83 (1H, 2d, J 3.1 und 5.0 Hz, C₂H), 7.32 (5H, s, C₆H₅) ppm]. Das Sulfinamid (3.36 g, 10 mMol) wird der Reaktion mit Kaliumpermanganat wie im Beispiel 5 angeführt, unterworfen. Das ölige Produkt (2.5 g wird mit Ether (30 ml) versetzt und die erhaltene gelatinöse Masse wird bei Raumperatur 8 Stunden gerührt. Das abgeschiedene kristallinische Produkt wird abgesaugt und mit Ether gewaschen.
Schmp. 111-130°C, Rf 0.10 (Benzol:EtOAc-3:1);
IR (KBr): 3300 vs, 1790vs, 1740vs, 1430s, 1330s, 1300s, 1120s, 1070s cm⁻¹;
¹H NMR (DMSO-d₆) δ: 2.96 (3H, dd, J 2.1 und 15.2 Hz, β C₃H), 3.29 (1H, dd, J 4.7 und 15.2 Hz, α C₃H), 4.23 (2H, d, J 5.9 Hz, NCH₂), 4.71 (1H, dd, J 2.1 und 4.7 Hz, C₂H), 7.33 (5H, s, C₆H₅), 7.96 (1H, t, J 5.9 Hz, SNH), 8.92 (1H, s, N₁H) ppm.

### b)

Das (2R) 1-(1'-Benzyloxycarbonyl-2'-methyl-prop-1'-enyl)-2-benzylaminosulfonyl-4-oxo-azetidin, hergestellt wie im Beispiel 11, wird analog dem Verfahren a), der Reaktion mit Kaliumpermanganat unterworfen, wobei ein identisches Produkt erhalten wird.

### BEISPIEL 13

### (2R) 1-(1'-Carboxyl-2'-methyl-prop-1'-enyl)-2-[(5'-methylisoxazol-3'-yl)aminosulfonyl]-4-oxo-azetidin

Die (2R) 1-(1'-Benzyloxycarbonyl-2'-methyl-prop-1'-enyl)-4-oxo-azetidin-2-sulfinsäure (3.23 g, 10 mMol) wird in Thionylchlorid (10 ml) gelöst. Die Lösung wird 1 Stunde bei 25°C gerührt. Der Thionylchloridüberschuß wird unter vermindertem Druck zu einem öligen Rückstand eingeengt. Der Rückstand wird in Methylenchlorid (50 ml) gelöst, mit 3-Amino-5-methyl-isoxazol (2.94 g, 30 mMol) versetzt und 3 Stunden bei Raumtemperatur gerührt. Das Reaktionsgemisch wird mit Wasser (50 ml) versetzt und mit 10%iger Salzsäure auf einen pH-Wert von 1.5 eingestellt. Die Schichten werden getrennt. Die organische Schicht wird erneut mit Wasser (50 ml) gewaschen. Die organische Schicht wird erneut mit Wasser (50 ml) versetzt und mit gesätigter Natriumhydrogenkarbonatlösung auf einen pH-Wert von 8.0 eingestellt. Die Schichten werden getrennt, die organische Schicht erneut mit Wasser gewaschen, über Natriumsulfat getrocknet und zur Trockne verdampft. Es wird eine Mischung (3.80 g, 92.6%) von epimeren (2R) 1-(1'-Benzyloxycarbonyl-2'-methyl-prop-1'-enyl)-2-[(5'-methyl-isoxazol-3'-yl)aminosulfinyl]-4-oxo-azetidinen mit R_{f}-Werten von 0.29 und 0.35 (CH₂Cl₂:EtOAc=2:1) erhalten, welche mittels Chromatographie an einer Silikagelsäule getrennt werden.
[Substanz mit einem R_{f}-Wert 0.29: IR (CH₂Cl₂) 1780vs, 1720m, 1620s, 1465s, 1360s, 1290m, 1215s, 1100s, 1080m cm⁻¹; ¹H NMR (CDCl₃) δ: 1.98 und 2.20 (6H, 2s, CMe₂), 2.34 (3H, s, Me-Isoxazol), 3.28 (1H, dd, J 4.7 und 15.5 Hz, α C₃H), 3.54 (1H, dd, J 2.6 und 15.5 Hz, β C₃H), 5.06 (1H, dd, J 2.6 und 4.7 Hz, C₂H), 5.19 (2H, s, CH₂Phenyl), 5.82 (1H, s, CH-Isoxazol), 7.33 (5H, s, C₆H₅), 8.40 (1H, s, NH) ppm; Substanz mit einem Rf-Wert von 0.35; IR (CH₂Cl₂) 1780vs, 1725m, 1630s, 1470s, 1360m, 1290m, 1220s, 1100s cm⁻¹;
¹H NMR (CDCl₃) δ: 2.09 und 2.24 (6H, 2s, CMe₂), 2.36 (3H, s, Me-Isoxazol), 3.04 (1H, dd, J 2.9 und 15.5 Hz, β C₃H), 3.30 (1H, dd, J 5.0 und 15.5 Hz, α C₃H), 5.11 (1H, dd, J 2.9 und J 5.0 Hz, C₂H), 5.21 (2H, s, CH₂Ph), 5.80 (1H, s, CH-Isoxazol), 7.26 (5H, s) ppm].

Die Lösung, bestehend aus Sulfinamid (4.03 g, 10 mMol), m-Chlorperbenzoesäure (4.30 g, 25 mMol) und Ethylacetat (50 ml) wird 24 Stunden bei Raumtemperatur gerührt. Nach beendeter Reaktion wird wäßrige Natriumthiosulfatlösung (15 mMol) zugetropft und weitere 30 Minuten gerührt. Das Reaktionsgemisch mit 5%iger wäßrigen Natriumhydrogenkarbonatlösung auf einen pH-Wert von 8.5 eingestellt und die Schichten getrennt. Der wäßrige Teil wird mit Natriumchlorid gesätigt und mit Ethylacetat extrahiert (3 x 30 ml). Die vereinigten organischen Schichten werden mit Salzwasser (30 ml) gewaschen, über Natriumsulfat getrocknet und zur Trockne verdampft. Es werden (3.5 g, 79%) des Rohproduktes in Form des Natriumsalzes erhalten. Durch Auflösung des Rohproduktes in Wasser und die Ansäuerung mit einer 10%igen Salzsäurelösung bis zu einem pH-Wert von 2.0 wird das (2R) 1-(1'-Benzyloxycarbonyl-2'-methyl-prop-1'-enyl)-2-[(5'-methyl-isoxazol-3'-yl)amino-sulfonyl]-4-oxo-azetidin erhalten [Schmp. 141-143°C (Kristall. aus Ether); R_{f} 0.40 (CH₂Cl₂:EtOAC-2:1); IR (KBr): 3200s, 1790vs, 1720vs, 1620s, 1465s, 1395s, 1300s, 1175s cm⁻¹; ¹H NMR (CDCl₃) δ: 2.02 und 2.14 (6H, 2s, CMe₂), 2.37 (3H, s, Me-Isoxazol), 3.32 (2H, d, J 3.5 Hz, α C₃H und β C₃H), 5.10 (2H, s, CH₂Ph), 5.28 (1H, t, J 3.5 Hz, C₂H), 6.10 (1H, s, CH-Isoxazol), 7.25 (5H, s, C₆H₅) ppm.
Anal.: C₁₉H₂₁N₃O₆S; Gef.: C, 54.21; H, 5.34; N, 9.96, S, 8.23%; Ber.: C, 54.41, H, 5.05, N, 10.02, S, 7.64%].

Das erhaltene Sulfonamid (419 mg, 1 mMol) wird hydriert und bearbeitet, wie im Beispiel 6 angeführt wurde. Es werden 227 mg (69%) erhalten:
R_{f} 0.91 (n-BuOH:HAc:H₂O=4:1:1);
IR (KBr): 3600-3000b, 3175s, 1795s, 1760s, 1680vs, 1620vs, 1520m, 1470vs, 1400vs, 1310s, 1270m, 1180vs, 1080m, 1045m, cm⁻¹;
¹H NMR (DMSO-d₆) δ: 1.89 und 2.13 (6H, 2s, CMe₂), 2.35 (3H, s, Me-Isoxazol), 3.21 (1H, dd, J 1.8 und 15.3 Hz, β C₃H), 3.55 (1H, dd, J 4.5 und 15.3 Hz, α C₃H), 5.34 (1H, dd, J 1.8 und 4.5 Hz, C₂H), 6.07 (1H, s, CH-Isoxazol), 11.32 (1H, bs, NH), 13.05 (1H, b, COOH) ppm.

### BEISPIEL 14

### (2R) 1-(1'-Benzyloxycarbonyl-2'-methyl-prop-1'-enyl)-2-[(3',4'-dimethyl-isoxazol-5'-yl)aminosulfonyl]-4-oxo-azetidin

Die (2R) 1-(1'-Benzyloxycarbonyl-2'-methyl-prop-1'-enyl)-4-oxoazetidin-2-sulfinsäure (3.23 g, 10 mMol) wird in Thionylchlorid (10 ml) gelöst. Die Lösung wird 1 Stunde bei 25°C gerührt. Das überschüssige Thionylchlorid wird unter vermindertem Druck zu einem öligen Rückstand eingeengt. Der eingeengte Rückstand wird in Methylenchlorid (50 ml) gelöst, mit 5-Amino-3,4-dimethyl-isoxazol (3.78 g, 30 mMol) versetzt und wie im Beispiel 13 angeführt, bearbeitet. Es wird ein Gemisch (3.20 g, 76.7 %) aus epimeren (2R) 1-(1'-Benzyloxycarbonyl-2'-methyl-prop-1'-enyl)-2-[(3',4'-dimethyl-isoxazol-5'-yl)amino-sulfinyl]-4-oxo-azetidinen mit R_{f}-Werten von 0.31 und 0.38 (CH₂Cl₂:EtOAc=2:1) erhalten, welches mittels Chromatographie an einer Silikagelsäule getrennt wird [Substanz mit einem Rf-Wert von 0.31: IR (KBr): 1790vs, 1730m 1710s, 1650s, 1370m, 1300m, 1225vs, 1100m cm⁻¹; ¹H NMR (CDCl₃) δ: 1.82, 1.97, 2.16, 2.20 (12H, 4s, 4Me), 3.17 (1H, d, J 4.1 Hz, α C₃H), 3.45 (1H, d, J 3.2 Hz, β C₃H), 5.04 (1H, dd, J 3.2 und 4.1 Hz, C₂H), 5.24 (2H, s, CH₂Ph), 7.35 (5H, s, C₆H₅), 7.87 (1H, s, NH) ppm; Substanz mit einem Rf-Wert von 0.38; IR (KBr): 1785vs, 1730s, 1700s, 1650s, 1370m, 1300m, 1230s, 1100s cm⁻¹; ¹H NMR (CDCl₃) δ: 1.81, 2.10, 2.17, 2.24 (12H, 4s, 4Me), 3.09 (1H, d, J 2.9 Hz, β C₃H), 3.34 (1H, d, J 5.0 Hz, α C₃H), 5.05 (1H, dd, J 2.9 und 5.0 Hz, C₂H), 5.20-5.30 (3H, m, CH₂Phenyl und NH), 7.35 (5H, s, C₆H₅), ppm]. Die Lösung, bestehend aus Sulfinamid (4.17 g, 10 mMol), meta-Chlorbenzoesäure (4.3 g, 25 mMol) und Ethylacetat (50 ml), wird bei Raumtemperatur 24 Stunden gerührt und wie im Beispiel 13 beschrieben wurde, bearbeitet. Es werden 3.2 g (70%) des Produktes in Form eines Natriumsalzes mit einem Schmp. von 160°C erhalten:
R_{f} 0.44 (EtOAc);
IR (CH₃Cl₂): 1790vs, 1730m, 1365s, 1220s, 1170s, 1070m cm⁻¹;
¹H NMR (CDCl₃) δ: 1.90, 2.03, 2.21, 2.23 (12H, 4s, 4Me), 3.29 (2H, d, J 4.0 Hz, α C₃H und β C₃H), 5.17 (1H, t, J 4.0 Hz, C₂H), 5.24 (2H, s, CH₂Phenyl), 7.35 (5H, s, C₆H₅) ppm.

### BEISPIEL 15

### (2R) 1-(1'-Benzyloxycarbonyl-2'-methyl-prop-1'-enyl)-2-[(2'-phenyl-pyrazol-3'-yl)aminosulfonyl]-4-oxo-azetidin

Die (2R) 1-(1'-Benzyloxycarbonyl-2'-methyl-prop-1'-enyl)-4-oxo-azetidin-2-sulfinsäure (10 mMol) wird in Thionylchlorid (3.23 g, 10 ml) gelöst und wie im Beispiel 13 beschrieben, bearbeitet, wobei an Stelle von 3-Amino-5-methyl-Isoxazol das 2-Phenyl-3-amino-pyrazol (4.77 g, 30 mMol) zugegeben wird. Es wird ein Gemisch (4.04 g, 86%) von epimeren (2R) 1-(1'-Benzyloxycarbonyl-2'-methyl-prop-1'-enyl)-2-[(2'-phenyl-pyrazol-3'-yl)aminosulfinyl]-4-oxo-azetidinen mit Rf-Werten von 0.44 und 0.50 (CH₂Cl₂:EtOAc=2:1) erhalten, welche mittels Chromatographie an einer Silikagelsäure getrennt werden [Substanz mit einem Rf-Wert von 0.44: IR (KBr): 1780vs, 1720s, 1600m, 1500s, 1455m, 1360m, 1290m, 1215vs, 1080m cm⁻¹; ¹H NMR (CDCl₃) δ: 1.93 und 2.16 (6H, 2s, CMe₂), 2.96 (1H, dd, J 4.9 und 15.3 Hz, α C₃H), 3.07 (1H, dd, J 2.5 und 15.3 Hz, β C₃H), 4.79 (1H, dd, J 2.5 und 4.9 Hz, C₂H), 5.14 (2H, s, CH₂Phenyl), 6.22 und 7.57 (2H, 2d, J 1.9 Hz, =CH-CH=), 7.26-7.45 (10H, m, 2C₆H₅) ppm; Substanz mit einem Rf-Wert von 0.50: IR (KBr): 1780vs, 1720s, 1600m, 1500s, 1455m, 1385m, 1360m, 1290m, 1220s, 1100s, 1070m cm⁻¹; ¹H NMR (CDCl₃) δ: 2.01 und 2.13 (6H, 2s, CMe₂), 2.80 (1H, dd, J 2.3 und 15.5 Hz, β C₃H), 2.92 (1H, dd, J 5.3 und 15.5 Hz, α C₃H), 4.80 (2H, b, C₂H), 5.09 und 5.18 (2H, ABq, J 12.4 Hz, CH₂Phenyl), 6.21 und 7.58 (2H, 2d, J 1.6 Hz =CH-CH=), 7.20-7.40 (10H, m, 2C₆H₅) ppm. Die Lösung bestehend aus Sulfinamid (4.64 g, 10 mMol), m-Chlorperbenzoesäure (4.3 g, 25 mMol) und Ethylacetat (50 ml) wird 24 Stunden bei Raumtemperatur gerührt und wie im Beispiel 13 beschrieben wurde, bearbeitet. Es wird 3.4 g (67%) des Produktes in Form des Natriumsalzes erhalten:
R_{f} 0.47 (EtOAc);
IR (CH₃Cl₃): 3340w, 1785s, 1725m, 1700m, 1500s, 1390s, 1360s, 1290m, 1215s, 1170s, 1075m cm⁻¹;
¹H NMR (CDCl₃) δ: 1.96 und 2.16 (6H, 2s, CMe₂), 2.88 (1H, dd, J 5.2 und 15.5 Hz, α C₃H), 3.00 (1H, dd, J 2.3 und 15.5 Hz, β C₃H), 4.92 (1H, dd, J 2.3 und 5.2, C₂H), 5.07 und 5.18 (2H, ABq, J 12.1 Hz, CH₂Phenyl), 6.20 und 7.56 (2H, 2d, J 1.7 Hz, =CH-CH=), 7.20-7.40 (10H, m, 2C₆H₅) ppm.

### BEISPIEL 16

### (2R,3R) 1-(1'-m-Methylbenzyloxycarbonyl-2'-methyl-prop-1'-enyl-2-[(5'-methyl-isoxazol-3'-yl)aminosulfonyl]-3-[(3'-o-chlorphenyl-5'-methyl-isoxazol-4'-yl)carboxamido]-4-oxoazetidin

Das epimere Gemisch der Sulfinamide (2.24 g), welches gemäß dem im Beispiel 7 beschriebenen Verfahren hergestellt wurde, wird in Methylenchlorid (10 ml) gelöst und mit Triethylamin (0.48 ml) 2 Stunden bei Raumtemperatur gerührt. Es wird mit 0.1 N Salzsäure (5 ml) und anschließend mit Wasser (10 ml) extrahiert und der organische Extrakt wird getrocknet (Na₂SO₄), filtriert und im Vakuum eingeengt. Es werden 2.23 g (99.5%) des Rohproduktes erhalten, aus welchem mittels Chromatographie an einer Silikagelsäule und Eluierung mit einem Lösungsmittelgemisch CH₂CL₂ : EtOAc das (2R, 3R) 1-(1'-m-Methylbenzyloxycarbonyl-2'-methyl-prop-1'-enyl)-2-[(5'-methyl-isoxazol-3'-yl)aminosulfinyl]-3-[(3'-o-chlorphenyl-5'-methyl-isoxazol-4'-yl)carboxamido]-4-oxo-azetidin [Rf 0.21 (CH₂Cl₂:EtOAC-4:1); Schmp. 94-96°C; ¹H NMR (CDCl₃) δ: 1.95 und 2.22 (6H, 2s, CMe₂), 2.33 und 2.35 (6H, 2s, 2Me-Isoxazol), 2.77 (3H, s, Me-Phenyl), 4.96 (1H, d, J 4.5 Hz, C₂H), 5.13 (2H, bs, OCH₂), 5.61 (1H, dd, J 4.5 und 8.5 Hz, C₃H), 5.72 (1H, s, CH-Isoxazol), 6.69 (1H, d, J 8.5 Hz, CONH), 7.04-7.55 (8H, m, 2C₆H₄) ppm], erhalten wird, welches anschließend mit Wasserstoffperoxid wie im Beispiel 1 beschrieben, oxidiert wird. Es wird das Sulfonamid in 85.8%-iger Ausbeute erhalten:
R_{f} 0.55 (CH₂Cl₂:MeOH-10:1);
IR (Film) 3410w, 3160vw, 3070vw, 1795vs, 1735w, 1685bs, 1620s, 1640m, 1580bs, 1420w, 1300m, 1270m, 1220m, 1170m, 1120m, 1060m, 770m, 740m cm⁻¹;
¹H NMR (CDCl₃) δ: 1.84 und 2.11 (6H, 2s, CMe₂), 2.26 und 2.33 (6H, 2s, 2Me-Isoxazol), 2.72 (3H, s, Me-Phenyl), 5.01 (2H, bs, OCH₂), 5.36 (1H, d, J 5.0 Hz, C₂H), 5.71-5.92 (2H, m, C₃H und CH-Isoxazol), 6.41 (1H, d, J 10 Hz, CONH), 7.01-7.62 (8H, m, 2C₆H₄) ppm.

### BEISPIEL 17

### (2R,3R) 1-(1'-Carboxyl-2'-methyl-prop-1'-enyl)-2-[(5'-methylisoxazol-3'-yl)aminosulfonyl]-3-phthalimido-4-oxo-azetidin

### a)

Das gemäß Beispiel 4 erhaltene (2R,3R) 1-(1'-p-Nitrobenzyloxycarbonyl-2'-methyl-prop-2'-enyl)-2-[(5'-methyl-isoxazol-3'-yl)aminosulfinyl]-3-phthalimido-4-oxo-azetidin wird in Methylenchlorid gelöst und mit Triethylamin gerührt, wobei das (2R,3R) 1-(1'-p-Nitrobenzyloxycarbonyl-2'-methyl-prop-1'-enyl)-2'-[(5'-methyl-isoxazol-3'-yl)aminosulfinyl]-3-phthalimido-4-oxo-azetidin [¹H NMR (CDCl₃) δ: 2.14 (6H, bs, CMe₂), 2.31 (3H, s, Me-Isoxazol), 5.08 und 5.23 (2H, ABq, J 13.5 Hz, OCH₃), 5.58 (1H, d, J 5.4 Hz, C₂H), 5.71 (1H, bs, CH-Isoxazol), 6.0 (1H, d, J 5.4 Hz, C₃H), 7.45 und 8.16 (4H, 2d, J 9.0 Hz, C₆H₄NO₂), 7.70-7.94 (4H, m, Phthalimido) ppm] erhalten wird, welches bei der Oxidation mit Wasserstoffperoxid, wie im Beispiel 1 angeführt, das (2R,3R) 1-(1'-p-Nitrobenzyloxycarbonyl-2'-methyl-prop-1'-enyl)-2-[(5'-methyl-isoxazol-3'-yl)-aminosulfonyl]-3-phthalimido-4-oxo-azetidin [R_{f} 0.60 (CH₂Cl₂:MeOH-9:1); IR (KBr): 1790bs, 1730vs, 1615m, 1520m, 1465m 1385s, 1350m, 1290w cm⁻¹; ¹H NMR (CDCl₃) 6: 2.12 und 2.26 (6H, 2s, CMe₂), 2.32 (3H, s, Me-Isoxazol), 5.21 (2H, bs, OCH₂), 5.73 (1H, d, J 5.4 Hz, C₂H), 5.83 (1H, d, J 5.4 Hz, C₃H), 6.05 (1H, bs, CH-Isoxazol), 7.50 und 8.18 (4H, 2d, J 9.0 Hz, C₆H₄NO₂), 7.60-7.86 (4H, m, Phthalimido) ppm] ergibt. Das Sulfonamid (840 mg, 1.38 mMol) wird in
Methanol (25 ml) gelöst, hydriert und bearbeitet, wie im Beispiel 6 angeführt wurde. Mittels Rühren des organischen Extraktes bei Raumtemperatur wird die Säure (490 mg, 75%) mit einem Schmp. von 160-165°C abgeschieden: IR (KBr) 3515m 3200m, 1795s, 1780s, 1735vs, 1685m, 1625m, 1525w, 1475m, 1415m, 1390vs, 1310w, 1180m cm⁻¹;
¹H NMR (DMSO-d₆) δ: 2.17 (6H, s, CMe₂), 2.27 (3H, s, Me-Isoxazol), 3.31 (2H, bs, SNH, COOH, HOH), 5.68 (1H, d, J 4.9 Hz, C₂H), 5.82 (1H, d, J 4.9 Hz, C₃H), 5.89 (1H, s, CH-Isoxazol), 7.92 (4H, s, Phthalimido) ppm.

### b)

Das gemäß Beispiel 4 hergestellte Sulfonamid (0.63 g) wird in Methylenchlorid (10 ml) gelöst und mit Triethylamin (0.1 g) bei 10°C im Laufe von 5 Stunden gerührt. Durch Chromatographie an einer Silikagelsäure wird aus dem eingeengten Rückstand 0.57 g Sulfonamid, welches mit dem ad a) beschriebenen identisch ist und welches durch Hydrogenolyse die Säure liefert, erhalten.

### BEISPIEL 18

### (2R,3R) 1-(1'-p-Nitrobenzyloxycarbonyl-2'-methyl-prop-1'-enyl)-2-[(5'-methyl-isoxazol-3'-yl)ominosulfonyl]-3-omino-4-oxo-azetidin

Das gemäß Beispiel 17 hergestellte Sulfonamid wird der Reaktion mit Natrumsulfid, dann mit Dicyclohexylcarbodiimid und abschließend mit Methylhydrazin wie in Croat. Chem. Acta **49** (1977) 779 beschrieben worden ist, unterworfen. Es wird ein schaumartiges Produkt, welches eine Ninhydrin-positive Reaktion ergibt, gewonnen:
R_{f} 0.48 (CH₂Cl₂:MeOH-9:1)
IR (KBr) 1785s, 1735m, 1710m, 1620m, 1525s, 1465w, 1400w, 1150s, 1300w, 1275w, 1220m, 1165m cm⁻¹;
¹H NMR (CDCl₃) δ: 2.13 und 2.25 (6H, 2s, CMe₂), 2.39 (3H, s, Me-Isoxazol), 4.66 (1H, d, J 5.2 Hz, C₂H), 5.21 (1H, d, J 5.2 Hz, C₃H), 5.22 und 5.29 (2H, ABq, J 12.8 Hz, OCH₂), 6.12 (1H, s, CH-Isoxazol), 7.49 und 8.22 (4H, 2d, J 8.5 Hz, C₆H₄NO₂) ppm.

### BEISPIEL 19

### (2R,3R) 1-(1'-Carbonyl-2'-methyl-prop-1'-enyl)-2-methylaminosulfonyl-3-o-methyl-aminocarbonylphenylcarboxamido-4-oxo-azetidin

Der (5R,6R) 6-Phthalimidopenicillanat-sulfoxid-p-nitrobenzylester (1.5 g, 3 mMol) wird der Reaktion mit N-Chlorsukzinimid (0.4 g, 3 mMol), wie im Beispiel 1 angeführt, unterworfen, wonach die Reaktionslösung auf 5°C abgekühlt und mit Methylamin (1 ml) in Toluol (4 ml) versetzt wird. Das Reaktionsgemisch wird 3 Stunden bei 5°C gerührt und im Vakuum eingeengt. Der Rückstand wird in Methylenchlorid (15 ml) suspendiert; der unlösliche Teil wird abgesaugt und die Mutterlauge im Vakuum eingeengt. Es wird ein epimeres Gemisch aus 1.53 g (2R,3R) 1-(l'-p-Nitrobenzyloxycarbonyl-2'-methyl-prop-1'-enyl)-2-methylaminosulfinyl-3-o-methylaminocarbonylphenylcarboxamido-4-oxo-azetidin erhalten [IR (Film): 3250bm, 3065w, 2950w, 1780s, 1730sh, 1715vs, 1650m, 1605w, 1525s, 1350s, 1295m, 1220m, 1185s, 1060m, 855w, 820w, 740m cm⁻¹], welches mit Wasserstoffperoxid wie im Beispiel 1 angeführt wurde, oxidiert wird. Es werden 1.23 g (2R,3R) 1-(1'-p-Nitrobenzylozycarbonyl-2'-methyl-prop-1'-enyl)-2-methylaminosulfonyl-3-o-methylaminocarbonylphenylcarboxamido-4-oxo-azetidin erhalten [R_{f} 0.81 (CH₂Cl₂:MeOH=9:1); ¹H NMR (CDCl₃) δ: 2.15 und 2.31 (6H, 2s, CMe₂), 2.86 (3H, d, J 4,5 Hz, CONMe), 2.98 (3H, d, J 4.9 Hz, SO₂NMe), 5.16 (1H, d, J 4.9 Hz, C₂H), 5.31 und 5.38 (2H, ABq, J 13.2 Hz, OCH₂), 5.94 (1H, dd, J 4.9 und 10.4 Hz, C₃H), 6.38 (1H, m, SO₂NH), 6.97 (1H, d, H 10.4 Hz, CONH), 7,18 (1H, q, J 4.9 Hz, CONH), 7.44-7.52 (4H, m, OCC₆H₄CO), 7.54 und 8.25 (4H, 2d, J 8.7 Hz, C₆H₄NO₂) ppm], welche durch Hydrogenolyse, wie im Beispiel 17 beschrieben wurde, die Säure (0.68 g) mit einem Schmp. von 142°C (Zersetzung) liefert.
IR (KBr): 3410m, 3370m, 3170m, 2960m, 1785vs, 1720s, 1680s, 1615s, 1600w, 1560w, 1515m, 1440w, 1415w, 1375w, 1330s, 1315s, 1285s, 1210s, 1185w, 1155w, 1080m, 735m, 700m cm⁻¹;
¹H NMR (DMSO-d₆) δ: 2.01 und 2.19 (6H, 2s, CMe₂), 2.64 (3H, d, J 4.5 Hz, CONMe), 2.75 (3H, d, J 4.7 Hz, SO₂NMe), 5.24 (1H, d, J 5.0 Hz, C₂H), 5.65 (1H, dd, J 5.0 und 8.7 Hz, C₃H), 7.17 (1H, q, J 4.7 Hz, SO₂NH), 7.48-7.56 (4H, m, C₆H₄), 8.38 (1H, q, J 4.5 Hz, CONH), 9.07 (1H, d, J, 8.7 Hz, CONH) ppm.

### BEISPIEL 20

### (2R,3R) 1-(1'-Carboxyl-2'-methyl-prop-1'-enyl)-2-benzylaminosulfonyl-3-phenyl-acetamido-4-oxo-azetidin

Der (5R,6R) 6-Phenylacetamidopenicillanat-sulfoxid-p-nitrobenzylester (3.0 g, 6.2 mMol) wird der Reaktion mit N-Chlorsukzinimid (1.0 g, 7.5 mMol) wie im Beispiel 1 angeführt, unterworfen, wonach mit Benzylamin (1.3 ml, 12.4 mMol) versetzt wird und das Reaktionsgemisch 2 Stunden bei 5°C gerührt wird. Die Toluollösung wird dekantiert, mit Wasser gewaschen, getrocknet und im Vakuum eingeengt. Durch Zugabe von Ethylacetat erhält man das (2R,3R) 1-(1'-p-Nitrobenzyloxycarbonyl-2'-methyl-prop-2'-enyl)-2-benzylamino-sulfinyl-3-phenyl-acetamido-4-oxo-azetidin, welches abgesaugt (1.82 g) [¹H NMR (CDCl₃) δ: 1.91 (3H, s, Me), 3.48 (2H, s, CH₂CO), 3.95-4.32 (3H, m, SNHCH₂), 4.80-5.14 (4H, m, NCHCO=CH₂, C₂H), 5.26 (2H, bs, OCH₂), 5.87 (1H, dd, J 5.0 und 9.8 Hz, C₃H), 6.52 (1H, d, J 9.8 Hz, CONH), 7.12-7.37 (10H, m 2C₆H₅), 7.47 und 8.22 (4H, 2d, J 8.8 Hz, C₆H₄NO₂) ppm], mit Triethylamin in Methylenchlorid in das (2R,3R) 1-(1'-p-Nitrobenzyloxycarbonyl-2'-methyl-prop-1'-enyl)-2-benzylamino-sulfinyl-3-phenylacetamido-4-oxo-azetidin (1.66 g) isomerisiert und mit Wasserstoffperoxid wie im Beispiel 1 angeführt, oxidiert wird. Es wird das (2R,3R) 1-(1'-p-Nitrobenzyloxycarbonyl-2'-methyl-prop-1'-enyl)-2-benzylaminosulfonyl-3-phenylacetamido-4-oxo-azetidin (1.57 g) erhalten: R_{f} 0.60 (CH₂Cl₂:EtOAC=4:1); [¹H NMR (CDCl₃) δ: 2.06 in 2.24 (6H, 2s, CMe₂), 3.59-3.98 (5H, m, CH₂CO, SNHCH₂), 4.67 (1H, d, J 5.0 Hz, C₂H), 5.21 (2H, bs, OCH₂), 5.73 (1H, dd, J 5.0 und 10.3 Hz, C₃H), 6.68 (1H, d, J 10.3 Hz, CONH), 7.00-7.36 (10H, m, 2C₆H₅), 7.42 und 8.19 (4H, 2d, J 8.8 Hz, C₆H₄NO₂) ppm], welches anschließend wie im Beispiel 6 angeführt, hydriert wird. Es werden 0.91 g des Produktes isoliert:
R_{f} 0.38 (CH₂Cl₂:MeOH=4:1);
IR (Film): 3500-2300bm, 1785s, 1740-1600bs, 1525m, 1340s, 1270m, 1210m, 1160s, 1070m, 740m, 705s cm⁻¹;
¹H NMR (CDCl₃) δ: 2.07 und 2.25 (6H, 2s, CMe₂), 3.56 und 3.64 (2H, ABq, J 14.8 Hz, CH₂CO), 3.98-4.02 (3H, m, SNHCH₂), 4.94 (1H, d, J 5.2 Hz, C₂H), 5.84 (1H, dd, J 5.2 und 10.3 Hz, C₃H), 6.77 (1H, d, J 10.3 Hz, CONH), 7.11-7.37 (10H, m, 2C₆H₅) ppm.

### BEISPIEL 21

### (2R,3R) 1-(1'-Carboxyl-2'-methyl-prop-1'-enyl)-2-methylaminosulfonyl-3-phenylacetamido-4-oxo-azetidin

Der (5R,6R) 6-Phenylacetamidopenicillanat-sulfoxid-p-nitrobenzylester (3.0 g, 6.2 mMol) wird der Reaktion mit N-Chlorsukzinimid (1.0 g, 7.5 mMol), wie im Beispiel 1 angeführt, unterworfen, wonach mit Methylamin (1 ml) versetzt wird und das Reaktionsgemisch 2 Stunden bei 5°C gerührt wird. Der Niederschlag wird abgesaugt, die Mutterlauge mit Wasser gewaschen, getrocknet und im Vakuum eingeengt. Die Chromatographie an einer Silikagelsäule ergibt das (2R,3R) 1-(1'-p-Nitrobenzyloxycarbonyl-2'-methyl-prop-1'-enyl)-2-methylamino-sulfinyl-3-phenylacetamido-4-oxo-azetidin (1.63 g) ¹H NMR (CDCl₃) δ: 2.12 und 2.26 (6H, 2s, CMe₂), 2.47 (3H, d, J 5.4 Hz, NMe), 3.63 (2H, ABq, J 14.9 Hz, CH₂CO), 4.68 (1H, d, J 5.0 Hz, C₂H), 5.28 (2H, s, OCH₂), 5.80 (1H, dd, J, 5.0 und 9.9 Hz, C₃H), 7.19 (1H, d, J 9.9 Hz, CONH), 7.26-7.38 (5H, m, C₆H₅), 7.50 und 8.24 (4H, 2d, J 8.7 Hz, C₆H₄NO₂) ppm], welches anschließend mit Wassserstoffperoxid wie im Beispiel 1 angeführt, oxidiert wird. Es wird das (2R,3R) 1-(1'-p-Nitrobenzyloxycarbonyl-2'-methyl-prop-1'-enyl)-2-methylamino-sulfonyl-3-phenyl-acetamido-4-oxo-azetidin (0.98 g) erhalten [IR (KBr): 3460-3140bw, 1785s, 1730m, 1700-1675bm, 1610w, 1525s, 1350s, 1220m, 1155m, 1070m, 850w cm⁻¹; ¹H NMR (CDCl₃) δ: 2.09 und 2.26 (6H, 2s, CMe₂), 2.46 (3H, d, J 5.0 Hz, NMe), 3.17 (1H, q, J 5 Hz, SNH), 3.55 und 3.69 (2H, ABq, J 14.5 Hz, CH₂CO), 4.97 (1H, d, J 5.0 Hz, C₂H), 5.27 und 5.33 (2H, ABq, J 13.2 Hz, OCH₂), 5.80 (1H, dd, J, 5.2 und 10.3 Hz, C₃H), 6.58 (1H, d, J 10.3 Hz, CONH), 7.32-7.51 (5H, m, C₆H₅), 7.50 und 8.23 (4H, 2d, J 8.7 Hz, C₆H₄NO₂) ppm]. Das erhaltene Sulfonamid wird wie im Beispiel 6 angeführt, hydriert, und die durch Hydrogenolyse entstandene Säure wird isoliert (0.43 g):
IR (KBr): 3660-2440bm, 1785s, 1740-1620bm, 1335m, 1160m, 1075w, 740w, 705w cm⁻¹;
¹H NMR (CDCl₃) δ: 2.07 und 2.25 (6H, 2s, CMe₂), 2.53 (3H, d, J 4.5 Hz, NMe), 3.63 (2H, ABq, J 15.1 Hz, CH₂CO), 4.16 (1H, m, SNH), 5.24 (1H, d, J 5.2 Hz, C₂H), 5.88 (1H, dd, J 5.2 und 10.3 Hz, C₃H), 6.84 (1H, d, J 10.3 Hz, CONH), 7.26-7.40 (5H, m, C₆H₅) ppm.

### BEISPIEL 22

### (2R,3R) 1-(1'-Carboxyl-2'-methyl-prop-1'-enyl)-2-[(5'-methyl-isoxazol-3-yl)aminosulfonyl]-3-phenoxyacetamido-4-oxo-azetidin

Der (5R,6R) 6-Phenoxyacetamidopenicillanat-sulfoxid-p-nitrobenzylester (5.0 g, 10 mMol) wird der Reaktion mit N-Chlorsukzinimid (1.7 g, 13 mMol), wie im Beispiel 1 angeführt, unterworfen, wonach mit 3-Amino-5-methyl-isoxazol (4.1 g, 40 mMol) versetzt wird und das Reaktionsgemisch 2 Stunden bei 0°C gerührt wird. Die Toluollösung wird dekantiert, mit Wasser gewaschen, getrocknet und im Vakuum eingeengt. Es wird ein epimeres Gemisch (5.0 g, 83.6%), bestehend aus (2R, 3R) 1-(1'-p-Nitrobenzyloxycarbonyl-2'-methyl-prop-2'-enyl)-2-[(5'-methyl-isoxazol-3'-yl)-aminosulfinyl]-3-phenoxyacetamido-4-oxo-azetidinen erhalten; [das überwiegende Epimer mit einem Schmp. 196-198°C; IR (KBr): 3310w, 1775s, 1755m, 1665m, 1625m, 1520s, 1350s, 1240m, 1170m, 1100s, 915w, 855w, 755w cm⁻¹; ¹H NMR (CDCl₃) δ: 2.02 (3H, s, Me), 2.25 (3H, s, Me-Isoxazol), 4.45 und 4.54 (2H, ABq, J 15.1, OCH₂CO), 5.07 (1H, bs, NCHCO), 5.07 und 5.19 (2H, 2bs, =CH₂), 5.39 (1H, d, J 5.1 Hz, C₂H), 5.35 (2H, bs, OCH₂), 5.76 (1H, bs, CH-Isoxazol), 5.85 (1H, dd, J 5.0 und 9.0 Hz, C₃H), 6.91-7.36 (5H, m, C₆H₅), 7.49 (1H, d, CONH), 7.49 und 8.21 (4H, 2d, J 8.8 Hz, C₆H₄NO₂) ppm].

Das erhaltene epimere Gemisch wird mit Wasserstoffperoxid wie im Beispiel 1 angeführt, oxidiert und der Isomerisierung mit Triethylamin in Methylenchlorid unterworfen, wonach (3.5 g, 68.2%) des (2R,3R) 1-(1'-p-Nitrobenzyloxycarbonyl-2'-methyl-prop-1'-enyl)-2-(5'-methyl-isoxazol-3'-yl)-aminosulfonyl-3-phenoxyacetamido-4-oxo-azetidins erhalten werden. [R_{f} 0.67 CH₂Cl₂:MeOH.9:1); IR (KBr): 3410-2700m, 1795s, 1735m, 1700s, 1620m, 1525s, 1500m, 1465m, 1400m, 1355s, 1300m, 1220s, 1165m, 1065-1110m cm⁻¹; ¹H NMR (CDCl₃) δ: 2.11 und 2.20 (6H, 2s, CMe₂), 2.24 (3H, s, Me-Isoxazol), 4.35 und 4.50 (2H, ABq, J 15.1 Hz, OCH₂CO), 5.26 (2H, s, OCH₂), 5.59 (1H, d, J 5.2 Hz, C₂H), 6.00 (1H, s, CH-Isoxazol), 6.03 (1H, dd, J 5.2 und 10.5 Hz, C₃H), 6.90-7.35 (5H, s, OC₆H₅), 7.52 und 8.22 (4H, 2d, J 8.8 Hz, C₆H₄NO₂) ppm].

Das erhaltene Sulfonamid (0.56 g, 0.91 mMol) wird anschließend der Hydrogenolyse wie im Beispiel 6 angeführt, unterworfen und 0.23 g (52.5%) des Produktes isoliert:
R_{f} Wert 0.35 (CH₂Cl₂:MeOH=1.5:1.0)
IR(KBr) 3600-2400bm, 1795s, 1700bs, 1620m, 1500-1550s, 1235s, 1170s, 1065-1090m, 940m cm⁻¹;
¹H NMR (CDCl₃) δ: 2.05 und 2.19 (6H, 2s, CMe₂), 2.27 (3H, s, Me-Isoxazol), 4.50 und 4.59 (2H, ABq, J 15.0 Hz, OCH₂CO), 5.78 (1H, d, J 4.8 Hz, C₂H), 6.09 (1H, dd, J 4.8 und 10.5 Hz, C₃H), 6.23 (1H, s, CH-Isoxazol), 6.91-7.40 (5H, m, OC₆H₅), 7.78 (1H, d, J 10.5 Hz, CONH) ppm.

## Patentansprüche

1. 4-Oxo-azetidin-2-sulfonsäureamide und ihre Salze der allgemeinen Formel I 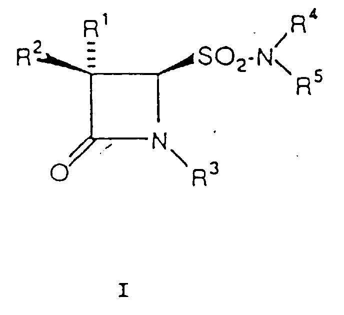 in welcher die Reste die folgenden Bedeutungen haben:
R¹ Wasserstoff, Halogen;
R² Wasserstoff, Halogen, NH₂, Phenyl-CH₂CONH, PhenylOCH₂CONH, Phthalimido, o-MeNHCOC₆H₄CONH, Isoxazolylcarbonylamino;
R³ Wasserstoff, Me₂C=C-COOMe, H₂C=C(Me)-CH-COOMe, Me₂C=C-COOCH₂Phenyl, H₂C=C(Me)-CH-COOCH₂C₆H₄NO₂-p, Me₂C=C-COOCH₂C₆H₄NO₂-p, Me₂C=C-COOCH₂C₆H₄Me-m, H₂C=C(Me)-CH-COOCH₂C₆H₄Me-m, Me₂C=C-COOH;
R⁴ Wasserstoff oder Natrium und
R⁵ Alkyl, Benzyl, Isoxazol oder Pyrazol.

2. Verbindung nach Anspruch 1, dadurch gekennzeichent, daß R¹ für Wasserstoff, R² für Wasserstoff, R³ für Me₂C=C-COOCH₂Phenyl, R⁴ für Wasserstoff und R⁵ für Phenyl-CH₂ stehen.

3. Verbindung nach Anspruch 1, dadurch gekennzeichnet, daß R¹ für Wasserstoff, R² für Wasserstoff, R³ für Me₂C=C-COOCH₂Phenyl, R⁴ für Natrium und R⁵ für Phenyl-CH₂ stehen.

4. Verbindung nach Anspruch 1, dadurch gekennzeichnet, daß R¹ für Wasserstoff, R² für Wasserstoff, R³ für Wasserstoff, R⁴ für Wasserstoff, und R⁵ Phenyl-CH₂ stehen.

5. Verbindung nach Anspruch 1, dadurch gekennzeichnet, daß R¹ für Brom, R² für Wasserstoff, R³ für Me₂C=C-COOCH₂Phenyl, R⁴ für Wasserstoff und R⁵ für Phenyl-CH₂ stehen.

6. Verbindung nach Anspruch 1, dadurch gekennzeichnet, daß R¹ für Wasserstoff, R² für Brom, R³ für Me₂C=C-COOCH₂Phenyl, R⁴ für Wasserstoff und R⁵ für Phenyl-CH₂ stehen.

7. Verbindung nach Anspruch 1, dadurch gekennzeichnet, daß R¹ für Brom, R² für Brom, R³ für Me₂C=C-COOCH₂Phenyl, R⁴ für Wasserstoff und R⁵ für Phenyl-CH₂ stehen.

8. Verbindung nach Anspruch 1, dadurch gekennzeichnet, daß R¹ für Brom, R² für Brom, R³ für Me₂C=C-COOCH₂Phenyl, R⁴ für Natrium und R⁵ für Phenyl-CH₂ stehen.

9. Verbindung nach Anspruch 1, dadurch gekennzeichnet, daß R¹ für Wasserstoff, R² für Wasserstoff, R³ für Me₂C=C-COOCH₂Phenyl, R⁴ für Wasserstoff und R⁵ für 5-Methyl-isoxazol-3-yl stehen.

10. Verbindung nach Anspruch 1, dadurch gekennzeichnet, daß R¹ für Wasserstoff, R² für Wasserstoff, R³ für Me₂C=C-COOCH₂Phenyl, R⁴ für Natrium und R⁵ für 5-Methyl-isoxazol-3-yl stehen.

11. Verbindung nach Anspruch 1, dadurch gekennzeichnet, daß R¹ für Brom, R² für Brom, R³ für Me₂C=C-COCCH₂Phenyl, R⁴ für Wasserstoff und R⁵ für 5-Methyl-isoxazol-3-yl stehen.

12. Verbindung nach Anspruch 1, dadurch gekennzeichnet, daß R¹ für Wasserstoff, R² für Wasserstoff, R³ für Me₂C=C-COOCH₂Phenyl, R⁴ für Wasserstoff und R⁵ für 3,4-Dimethyl-isoxazol-5-yl stehen.

13. Verbindung nach Anspruch 1, dadurch gekennzeichnet, daß R¹ für Wasserstoff, R² für Wasserstoff, R³ für Me₂C=C-COOCH₂Phenyl, R⁴ für Natrium und R⁵ für 3,4-Dimethyl-isoxazol-5-yl stehen.

14. Verbindung nach Anspruch 1, dadurch gekennzeichnet, daß R¹ für Wasserstoff, R² für Wasserstoff, R³ für Me₂C=C-COOCH₂Phenyl, R⁴ für Wasserstoff und R⁵ für 2-Phenyl-pyrazol-3-yl stehen.

15. Verbindung nach Anspruch 1, dadurch gekennzeichnet, daß R¹ für Wasserstoff, R² für Wasserstoff, R³ für Me₂C-C-COOCH₂Phenyl, R⁴ für Natrium und R⁵ für 2-Phenyl-pyrazol-3-yl stehen.

16. Verbindung nach Anspruch 1, dadurch gekennzeichnet, daß R¹ für Wasserstoff, R² für Phenyl-OCH₂CONH, R³ für Me₂C=C-COOMe, R⁴ für Wasserstoff und R⁵ für Phenyl-CH₂ stehen.

17. Verbindung nach Anspruch 1, dadurch gekennzeichnet, daß R¹ für Wasserstoff, R² für Phenyl-OCH₂CONH, R³ für H₂C-C(Me)CH-COOMe, R⁴ für Wasserstoff und R⁵ für 5-Methyl-isoxazol-3-yl stehen.

18. Verbindung nach Anspruch 1, dadurch gekennzeichnet, daß R¹ für Wasserstoff, R² für Phenyl-OCH₂CONH, R³ für Me₂C=C-COOMe, R⁴ für Wasserstoff und R⁵ für 5-Methyl-isoxazol-3-yl stehen.

19. Verbindung nach Anspruch 1, dadurch gekennzeichnet, daß R¹ für Wasserstoff, R² für Phthalimido, R³ für H₂C=C(Me)-CH-COOCH₂C₆H₄NO₂-p, R⁴ für Wasserstoff und R⁵ für 5-Methyl-isoxazol-3-yl stehen.

20. Verbindung nach Anspruch 1, dadurch gekennzeichnet, daß R¹ für Wasserstoff, R² für Phthalmido, R³ für Me₂C=C-COOCH₂C₆H₄NO₂-p, R⁴ für Wasserstoff und R⁵ für 5-Methyl-isoxazol-3-yl stehen.

21. Verbindung nach Anspruch 1, dadurch gekennzeichnet, daß R¹ für Wasserstoff, R² für Phenyl-CH₂CONH, R³ für Wasserstoff, R⁴ für Wasserstoff und R⁵ für Phenyl-CH₂ stehen.

22. Verbindung nach Anspruch 1, dadurch gekennzeichnet, daß R¹ für Wasserstoff, R² für Phenyl-CH₂CONH, R³ für Me₂C=C-COOCH₂C₆H₄NO₂-p, R⁴ für Wasserstoff und R⁵ für 5-Methyl-isoxazol-3-yl stehen.

23. Verbindung nach Anspruch 1, dadurch gekennzeichnet, daß R¹ für Wasserstoff, R² für Phenyl-CH₂CONH, R³ für Me₂C=C-COOH, R⁴ für Wasserstoff und R⁵ für Methyl-isoxazol-3-yl stehen.

24. Verbindung nach Anspruch 1, dadurch gekennzeichnet, daß R¹ für Wasserstoff, R² für Wasserstoff, R³ für Me₂C=C-COOH, R⁴ für Wasserstoff und R⁵ für Phenyl-CH₂ stehen.

25. Verbindung nach Anspruch 1, dadurch gekennzeichnet, daß R¹ für Wasserstoff, R² für 3-o-Chlorphenyl-5-methylisoxazol-4-yl, R³ für H₂C=C(Me)-CH-COOCH₂C₆H₄Me-m, R⁴ für Wasserstoff und R⁵ für 5-Methyl-isoxazol-3-yl stehen.

26. Verbindung nach Anspruch 1, dadurch gekennzeichnet, daß R¹ für Wasserstoff, R² für 3-o-Chlorphenyl-5-methylisoxazol-4-yl, R³ für Me₂C=C-COOCH₂C₆H₄Me-m, R⁴ für Wasserstoff und R⁵ für 5-Methyl-isoxazol-3-yl stehen.

27. Verbindung nach Anspruch 1, dadurch gekennzeichnet, daß R¹ für Wasserstoff, R² für Phthalmido, R³ für Me₂C=C-COOH, R⁴ für Wasserstoff und R⁵ für 5-Methylisoxazol-3-yl stehen.

28. Verbindung nach Anspruch 1, dadurch gekennzeichnet, daß R¹ für Wasserstoff, R² für Amino, R³ für Me₂C=C-COOCH₂C₆H₄NO₂-p, R⁴ für Wasserstoff und R⁵ für 5-Methyl-isoxazol-3-yl stehen.

29. Verbindung nach Anspruch 1, dadurch gekennzeichnet, daß R¹ für Wasserstoff, R² für o-MeNHCOC₆H₄CONH, R³ für Me₂C=C-COOCH₂C₆H₄NO₂-p, R⁴ für Wasserstoff und R⁵ für Methyl stehen.

30. Verbindung nach Anspruch 1, dadurch gekennzeichnet, daß R¹ für Wasserstoff, R² für o-MeNHCOC₆H₄CONH, R³ für H₂C=C(Me)-CH-COOCH₂C₆H₄NO₂-p, R⁴ für Wasserstoff und R⁵ für Methyl stehen.

31. Verbindung nach Anspruch 1, dadurch gekennzeichnet, daß R¹ für Wasserstoff, R² für o-MeNHCOC₆H₄CONH, R³ für Me₂C=C-COOH, R⁴ für Wasserstoff und R⁵ für Methyl stehen.

32. Verbindung nach Anspruch 1, dadurch gekennzeichnet, daß R¹ für Wasserstoff, R² für Phenyl-CH₂CONH, R³ für Me₂C=C-COOH, R⁴ für Wasserstoff und R⁵ für Phenyl-CH₂ stehen.

33. Verbindung nach Anspruch 1, dadurch gekennzeichnet, daß R¹ für Wasserstoff, R² für Phenyl-CH₂CONH, R³ für Me₂C=C-COOCH₂C₆H₄NO₂-p, R⁴ für Wasserstoff und R⁵ für Phenyl-CH₂ stehen.

34. Verbindung nach Anspruch 1, dadurch gekennzeichnet, daß R¹ für Wasserstoff, R² für Phenyl-CH₂CONH, R³ für Me₂C=C-COOCH₂C₆H₄NO₂-p, R' für Wasserstoff und R⁵ für Methyl stehen.

35. Verbindung nach Anspruch 1, dadurch gekennzeichnet, daß R¹ für Wasserstoff, R² für Phenyl-CH₂CONH, R³ für Me₂C=C-COOH, R⁴ für Wasserstoff und R⁵ für Methyl stehen.

36. Verbindung nach Anspruch 1, dadurch gekennzeichnet, daß R¹ für Wasserstoff, R² für Wasserstoff, R³ für Me₂C=C-COOH, R⁴ für Wasserstoff und R⁵ für 5-Methyl-isoxazol-3-yl stehen.

37. Verbindung nach Anspruch 1, dadurch gekennzeichnet, daß R¹ für Brom, R² für Brom, R³ für Me₂C=C-COOH, R⁴ für Wasserstoff und R⁵ für Phenyl-CH₂ stehen.

38. Verbindung nach Anspruch 1, dadurch gekennzeichnet, daß R¹ für Wasserstoff, R² für Phenyl-OCH₂CONH, R⁵ für Me₂C=C-COOH, R⁴ für Wasserstoff und R⁵ für 5-Methylisoxazol-3-yl stehen.

39. Verfahren zur Herstellung von 4-Oxo-azetidin-2-sulfonsäureamiden und deren Salzen der allgemeinen Formel I, in welcher die Reste die im Anspruch 1 angeführten Bedeutungen haben, dadurch gekennzeichnet, daß 4-Oxo-azetidin-2-sulfinsäureamide der allgemeinen Formel II, 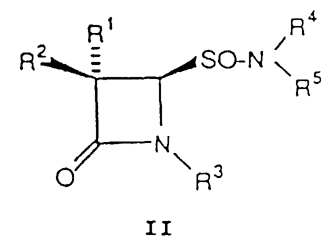 in welcher die Reste die folgenden Bedeutungen haben:
R¹ Wassserstoff oder Halogen;
R² Wasserstoff, Halogen, PhenylCH₂CONH, PhenylOCH₂CONH, Phthalimido, o-MeNHCOC₆H₄CONH, Isoxazolylcarbonylamino;
R³ Me₂C=C-COOMe, H₂C=C(Me)-CH-COOMe, Me₂C=C-COOCH₂Phenyl, H₂C=C(Me)-CH-COOCH₂C₆H₄NO₂-p, Me₂C=C-COOCH₂C₆H₄NO₂-p, Me₂C=C-COOCH₂C₆H₄Me-m, H₂C=C(Me)-CHCOOCH₂C₆H₄Me-m;
R⁴ Wasserstoff und
R⁵ Alkyl, Benzyl, Isoxazol oder Pyrazol.
in den für die in der organischen Chemie bekannten Oxidationen üblichen Mitteln, wie z.B. Wassserstoffperoxid, Peracetessigsäure, m-Chlorbenzoesäure und Kaliumpermanganat, in einem sauren oder neutralen, wäßrigen oder wäßrig-organischen Medium und bei einer Temperatur von 0 bis 100°C, oxidiert werden und anschließend die Abspaltung der Schutzgruppen, die Bearbeitung des Reaktionsgemisches und die Isolierung des Produktes auf übliche Weise ausgeführt werden.

40. Die Verwendung der Verbindung gemäß Anspruch 1 als Zwischenverbindung für die Synthese von beta-Lactamanalogen, insbesondere bizyklischer Systeme.

41. Die Verwendung der Verbindung gemäß Anspruch 1 als Wirkstoff für die Herstellung von Präparaten zur antimikrobiellen Therapie.

## Claims

1. 4-oxo-azetidine-2-sulfonic acid amides and their salts of the general formula I 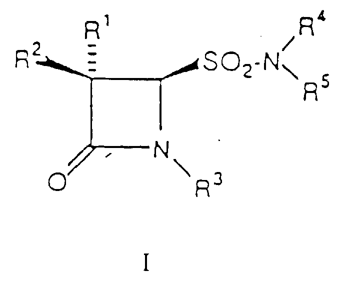 in which the groups are defined as follows:
R¹ represents hydrogen, halogen:
R² represents hydrogen, halogen, NH₂, phenyl-CH₂CONH, phenyl-OCH₂CONH. phthalimido, o-MeNHCOC₆H₄CONH, isoxazolylcarbonylamino;
R³ represents hydrogen, Me₂C=C-COOMe, H₂C=C(Me)-CH-COOMe, Me₂C=C-COOCH₂phenyl, H₂C=C(Me)-CH-COOCH₂C₆H₄NO₂-p, Me₂C=C-COOCH₂C₆H₄NO₂-p, Me₂C=C-COOCH₂C₆H₄Me-m, H₂C=C(Me)-CH-COOCH₂C₆H₄Me-m, Me₂C=C-COOH;
R⁴ represents hydrogen or sodium and
R⁵ represents alkyl, benzyl, isoxazole or pyrazole.

2. A compound according to Claim 1, characterised in that R¹ represents hydrogen. R² represents hydrogen, R³ represents Me₂C=C-COOCH₂phenyl, R⁴ represents hydrogen and R⁵ represents phenyl-CH₂.

3. A compound according to Claim 1, characterised in that R¹ represents hydrogen, R² represents hydrogen, R³ represents Me₂C=C-COOCH₂phenyl, R⁴ represents sodium and R⁵ represents phenyl-CH₂.

4. A compound according to Claim 1, characterised in that R¹ represents hydrogen, R² represents hydrogen, R³ represents hydrogen, R⁴ represents hydrogen and R⁵ represents phenyl-CH₂.

5. A compound according to Claim 1, characterised in that R¹ represents bromine, R² represents hydrogen, R³ represents Me₂C=C-COOCH₂Phenyl, R⁴ represents hydrogen and R⁵ represents phenyl-CH₂.

6. A compound according to Claim 1, characterised in that R¹ represents hydrogen, R² represents bromine, R³ represents Me₂C=C-COOCH₂phenyl, R⁴ represents hydrogen and R⁵ represents phenyl-CH₂.

7. A compound according to Claim 1, characterised in that R¹ represents bromine, R² represents bromine, R³ represents Me₂C=C-COOCH₂phenyl, R⁴ represents hydrogen and R⁵ represents phenyl-CH₂.

8. A compound according to Claim 1, characterised in that R¹ represents bromine, R² represents bromine, R³ represents Me₂C=C-COOCH₂phenyl, R⁴ represents sodium and R⁵ represents phenyl-CH₂.

9. A compound according to Claim 1, characterised in that R¹ represents hydrogen, R² represents hydrogen, R³ represents Me₂C=C-COOCH₂phenyl, R⁴ represents hydrogen and R⁵ represents 5-methyl-isoxazol-3-yl.

10. A compound according to Claim 1, characterised in that R¹ represents hydrogen, R² represents hydrogen, R³ represents Me₂C=C-COOCH₂phenyl, R⁴ represents sodium and R⁵ represents 5-methyl-isoxazol-3-yl.

11. A compound according to Claim 1, characterised in that R¹ represents bromine. R² represents bromine, R³ represents Me₂C=C-COOCH₂phenyl, R⁴ represents hydrogen and R⁵ represents 5-methyl-isoxazol-3-yl.

12. A compound according to Claim 1, characterised in that R¹ represents hydrogen, R² represents hydrogen, R³ represents Me₂C=C-COOCH₂phenyl, R⁴ represents hydrogen and R⁵ represents 3,4-dimethyl-isoxazol-5-yl.

13. A compound according to Claim 1, characterised in that R¹ represents hydrogen, R² represents hydrogen, R³ represents Me₂C=C-COOCH₂phenyl, R⁴ represents sodium and R⁵ represents 3,4-dimethyl-isoxazol-5-yl.

14. A compound according to Claim 1, characterised in that R¹ represents hydrogen, R² represents hydrogen, R³ represents Me₂C=C-COOCH₂phenyl, R⁴ represents hydrogen and R⁵ represents 2-phenyl-pyrazol-3-yl.

15. A compound according to Claim 1, characterised in that R¹ represents hydrogen, R² represents hydrogen, R³ represents Me₂C=C-COOCH₂phenyl, R⁴ represents sodium and R⁵ represents 2-phenyl-pyrazol-3-yl.

16. A compound according to Claim 1, characterised in that R¹ represents hydrogen, R² represents phenyl-OCH₂CONH, R³ represents Me₂C=C-COOMe, R⁴ represents hydrogen and R⁵ represents phenyl-CH₂.

17. A compound according to Claim 1, characterised in that R¹ represents hydrogen. R² represents phenyl-OCH₂CONH, R³ represents H₂C=C(Me)CH-COOMe, R⁴ represents hydrogen and R⁵ represents 5-methyl-isoxazol-3-yl.

18. A compound according to Claim 1, characterised in that R¹ represents hydrogen, R² represents phenyl-OCH₂CONH, R³ represents Me₂C=C-COOMe, R⁴ represents hydrogen and R⁵ represents 5-methyl-isoxazol-3-yl.

19. A compound according to Claim 1, characterised in that R¹ represents hydrogen, R² represents phthalimido, R³ represents H₂C=C(Me)-CH-COOCH₂C₆H₄NO₂-p, R⁴ represents hydrogen and R⁵ rcpresents 5-methyl-isoxazol-3-yl.

20. A compound according to Claim 1, characterised in that R¹ represents hydrogen, R² represents phthalimido, R³ represents Me₂C=C-COOCH₂C₆H₄NO₂-p, R⁴ represents hydrogen and R⁵ represents 5-methyl-isoxazol-3-yl.

21. A compound according to Claim 1, characterised in that R¹ represents hydrogen, R² represents phenyl-CH₂CONH, R³ represents hydrogen, R⁴ represents hydrogen and R⁵ represents phenyl-CH₂.

22. A compound according to Claim 1, characterised in that R¹ represents hydrogen, R² represents phenyl-CH₂CONH, R³ represents Me₂C=C-COOCH₂C₆H₄NO₂-p, R⁴ represents hydrogen and R⁵ represents 5-methyl-isoxazol-3-yl.

23. A compound according to Claim 1, characterised in that R¹ represents hydrogen, R² represents phenyl-CH₂CONH, R³ represents Me₂C=C-COOH, R⁴ represents hydrogen and R⁵ represents methyl-isoxazol-3-yl.

24. A compound according to Claim 1, characterised in that R¹ represents hydrogen, R² represents hydrogen, R³ represents Me₂C=C-COOH, R⁴ represents hydrogen and R⁵ represents phenyl-CH₂.

25. A compound according to Claim 1, characterised in that R¹ represents hydrogen, R² represents 3-o-chlorophenyl-5-methyl-isoxazol-4-yl, R³ represents H₂C=C(Me)-CH-COOCH₂C₆H₄Me-m, R⁴ represents hydrogen and R⁵ represents 5-methyl-isoxazol-3-yl.

26. A compound according to Claim 1, characterised in that R¹ represents hydrogen, R² represents 3-o-chlorophenyl-5-methyl-isoxazol-4-yl, R³ represents Me₂C=C-COOCH₂C₆H₄Me-m, R⁴ represents hydrogen and R⁵ represents 5-methyl-isoxazol-3-yl.

27. A compound according to Claim 1, characterised in that R¹ represents hydrogen, R² represents phthalimido, R³ represents Me₂C=C-COOH, R⁴ represents hydrogen and R⁵ represents 5-methyl-isoxazol-3-yl.

28. A compound according to Claim 1, characterised in that R¹ represents hydrogen, R² represents amino, R³ represents Me₂C=C-COOCH₂C₆H₄NO₂-p, R⁴ represents hydrogen and R⁵ represents 5-methyl-isoxazol-3-yl.

29. A compound according to Claim 1, characterised in that R¹ represents hydrogen, R² represents o-MeNHCOC₆H₄CONH, R³ represents Me₂C=C-COOCH₂C₆H₄NO₂-p, R⁴ represents hydrogen and R⁵ represents methyl.

30. A compound according to Claim 1, characterised in that R¹ represents hydrogen, R² represents o-MeNHCOC₆H₄CONH, R³ represents H₂C=C(Me)-CH-COOCH₂-C₆H₄NO₂-p, R⁴ represents hydrogen and R⁵ represents methyl.

31. A compound according to Claim 1, characterised in that R¹ represents hydrogen, R² represents o-MeNHCOC₆H₄CONH, R³ represents Me₂C=C-COOH, R⁴ represents hydrogen and R⁵ represents methyl.

32. A compound according to Claim 1, characterised in that R¹ represents hydrogen, R² represents phenyl-CH₂CONH, R³ represents Me₂C=C-COOH, R⁴ represents hydrogen and R⁵ represents phenyl-CH₂.

33. A compound according to Claim 1, characterised in that R¹ represents hydrogen, R² represents phenyl-CH₂CONH, R³ represents Me₂C=C-COOCH₂-C₆H₄NO₂-p, R⁴ represents hydrogen and R⁵ represents phenyl-CH₂.

34. A compound according to Claim 1, characterised in that R¹ represents hydrogen, R² represents phenyl-CH₂CONH, R³ represents Me₂C=C-COOCH₂-C₆H₄NO₂-p. R⁴ represents hydrogen and R⁵ represents methyl.

35. A compound according to Claim 1, characterised in that R¹ represents hydrogen, R² represents phenyl-CH₂CONH, R³ represents Me₂C=C-COOH, R⁴ represents hydrogen and R⁵ represents methyl.

36. A compound according to Claim 1, characterised in that R¹ represents hydrogen. R² represents hydrogen, R³ represents Me₂C=C-COOH, R⁴ represents hydrogen and R⁵ represents 5-methyl-isoxasol-3-yl.

37. A compound according to Claim 1, characterised in that R¹ represents bromine. R² represents bromine, R³ represents Me₂C=C-COOH, R⁴ represents hydrogen and R⁵ represents phenyl-CH₂.

38. A compound according to Claim 1, characterised in that R¹ represents hydrogen. R² represents phenyl-OCH₂CONH, R³ represents Me₂C=C-COOH, R⁴ represents hydrogen and R⁵ represents 5-methyl-isoxasol-3-yl.

39. A process for preparing 4-oxo-azetidine-2-sulfonic acid amides and their salts of the general formula I, in which the groups are defined in the same way as in Claim 1, characterised in that 4-oxo-azetidine-2-sulfinic acid amides of the general formula II 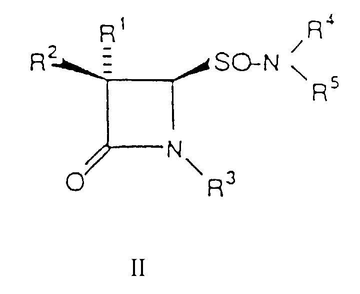 in which the groups are defined as follows:
R¹ represents hydrogen or halogen;
R² represents hydrogen, halogen, phenyl-CH₂CONH, phenyl-OCH₂CONH, phthalimido, o-MeNHCOC₆H₄CONH, isoxazolylcarbonylamino;
R³ represents Me₂C=C-COOMe, H₂C=C(Me)-CH-COOMe, Me₂C=C-COOCH₂phenyl, H₂C=C(Me)-CH-COOCH₂C₆H₄NO₂-p, Me₂C=C-COOCH₂C₆H₄NO₂-p, Me₂C=C-COOCH₂C₆H₄Me-m, H₂C=C(Me)-CH-COOCH₂C₆H₄Me-m;
R⁴ represents hydrogen and
R⁵ represents alkyl, benzyl, isoxazole or pyrazole
are oxidised in agents conventionally recognised for oxidations in organic chemistry such as e.g. hydrogen peroxide, peracetic acid, m-chlorobenzoic acid and potassium permanganate, in an acid or neutral, aqueous or aqueous/organic medium and at a temperature of 0 to 100°C, then the protective groups are removed, the reaction mixture processed and the product isolated in a conventional manner.

40. Use of the compound according to Claim 1 as an intermediate compound for the synthesis of beta-lactam analogues, in particular bicyclic systems.

41. Use of the compound according to Claim 1 as an active ingredient for producing preparations for antimicrobial treatment.

## Revendications

1. Amides de l'acide 4-oxo-azétidine-2-sulfonique, ainsi que leurs sels répondant à la formule générale I 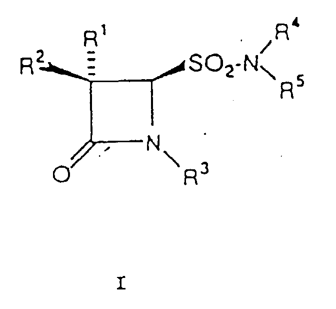 dans laquelle les radicaux ont les significations ci-après:
R¹ représente un atome d'hydrogène, un atome d'halogène;
R² représente un atome d'hydrogène, un atome d'halogène, un groupe NH₂, un groupe phényl-CH₂CONH, un groupe phényl-OCH₂CONH, un groupe phtalimido, un groupe o-MeNHCO-C₆H₄CONH, un groupe isoxazolylcarbonylamino;
R³ représente un atome d'hydrogène, un groupe Me₂C=C-COOOMe, un groupe H₂C=C(Me)-CH-COOMe, un groupe Me₂C=C-COO-CH₂phényle, un groupe H₂C=C(Me)-CH-COOCH₂C₆H₄NO₂-p, un groupe Me₂C=C-COOCH₂C₆H₄NO₂-p, un groupe Me₂C=C-COOCH₂C₆H₄Me-m, un groupe H₂C=C(Me)-CH-COOCH₂C₆H₄Me-m, un groupe Me₂C=C-COOH;
R⁴ représente un atome d'hydrogène ou un atome de sodium; et
R⁵ représente un groupe alkyle, un groupe benzyle, un groupe isoxazole ou un groupe pyrazole.

2. Composé selon la revendication 1, caractérisé en ce que R¹ représente un atome d'hydrogène, R² représente un atome d'hydrogène, R³ représente un groupe Me₂C=C-COOCH₂phényle, R⁴ représente un atome d'hydrogène et R⁵ représente un groupe phényl-CH₂.

3. Composé selon la revendication 1, caractérisé en ce que R¹ représente un atome d'hydrogène, R² représente un atome d'hydrogène, R³ représente un groupe Me₂C=C-COOCH₂phényle, R⁴ représente un atome de sodium et R⁵ représente un groupe phényl-CH₂.

4. Composé selon la revendication 1, caractérisé en ce que R¹ représente un atome d'hydrogène, R² représente un atome d'hydrogène, R³ représente un atome d'hydrogène , R⁴ représente un atome d'hydrogène et R⁵ représente un groupe phényl-CH₂.

5. Composé selon la revendication 1, caractérisé en ce que R¹ représente un atome de brome, R² représente un atome d'hydrogène, R³ représente un groupe Me₂C=C-COOCH₂phényle, R⁴ représente un atome d'hydrogène et R⁵ représente un groupe phényl-CH₂.

6. Composé selon la revendication 1, caractérisé en ce que R¹ représente un atome d'hydrogène, R² représente un atome de brome, R³ représente un groupe Me₂C=C-COOCH₂phényle, R⁴ représente un atome d'hydrogène et R⁵ représente un groupe phényl-CH₂.

7. Composé selon la revendication 1, caractérisé en ce que R¹ représente un atome de brome, R² représente un atome de brome, R³ représente un groupe Me₂C=C-COOCH₂phényle, R⁴ représente un atome d'hydrogène et R⁵ représente un groupe phényl-CH₂.

8. Composé selon la revendication 1, caractérisé en ce que R¹ représente un atome de brome, R² représente un atome de brome, R³ représente un groupe Me₂C=C-COOCH₂phényle, R⁴ représente un atome de sodium et R⁵ représente un groupe phényl-CH₂.

9. Composé selon la revendication 1, caractérisé en ce que R¹ représente un atome d'hydrogène, R² représente un atome d'hydrogène, R³ représente un groupe Me₂C=C-COOCH₂phényle, R⁴ représente un atome d'hydrogène et R⁵ représente un groupe 5-méthyl-isoxazol-3-yle.

10. Composé selon la revendication 1, caractérisé en ce que R¹ représente un atome d'hydrogène, R² représente un atome d'hydrogène, R³ représente un groupe Me₂C=C-COOCH₂phényle, R⁴ représente un atome de sodium et R⁵ représente un groupe 5-méthyl-isoxazol-3-yle.

11. Composé selon la revendication 1, caractérisé en ce que R¹ représente un atome de brome, R² représente un atome de brome, R³ représente un groupe Me₂C=C-COOCH₂phényle, R⁴ représente un atome d'hydrogène et R⁵ représente un groupe 5-méthyl-isoxazol-3-yle.

12. Composé selon la revendication 1, caractérisé en ce que R¹ représente un atome d'hydrogène, R² représente un atome d'hydrogène, R³ représente un groupe Me₂C=C-COOCH₂phényle, R⁴ représente un atome d'hydrogène et R⁵ représente un groupe 3,4-diméthyl-isoxazol-5-yle.

13. Composé selon la revendication 1, caractérisé en ce que R¹ représente un atome d'hydrogène, R² représente un atome d'hydrogène, R³ représente un groupe Me₂C=C-COOCH₂phényle, R⁴ représente un atome de sodium et R⁵ représente un groupe 3,4-diméthyl-isoxazol-5-yle.

14. Composé selon la revendication 1, caractérisé en ce que R¹ représente un atome d'hydrogène, R² représente un atome d'hydrogène, R³ représente un groupe Me₂C=C-COOCH₂phényle, R⁴ représente un atome d'hydrogène et R⁵ représente un groupe 2-phényl-pyrazol-3-yle.

15. Composé selon la revendication 1, caractérisé en ce que R¹ représente un atome d'hydrogène, R² représente un atome d'hydrogène, R³ représente un groupe Me₂C=C-COOCH₂phényle, R⁴ représente un atome de sodium et R⁵ représente un groupe 2-phényl-pyrazol-3-yle.

16. Composé selon la revendication 1, caractérisé en ce que R¹ représente un atome d'hydrogène, R² représente un groupe phényl-OCH₂CONH, R³ représente un groupe Me₂C=C-COOMe, R⁴ représente un atome d'hydrogène et R⁵ représente un groupe phényl-CH₂.

17. Composé selon la revendication 1, caractérisé en ce que R¹ représente un atome d'hydrogène, R² représente un groupe phényl-OCH₂CONH, R³ représente un groupe H₂C=C(Me)CH-COOMe, R⁴ représente un atome d'hydrogène et R⁵ représente un groupe 5-méthyl-isoxazol-3-yle.

18. Composé selon la revendication 1, caractérisé en ce que R¹ représente un atome d'hydrogène, R² représente un groupe phényl-OCH₂CONH, R³ représente un groupe Me₂C=C-COOMe, R⁴ représente un atome d'hydrogène et R⁵ représente un groupe 5-méthyl-isoxazol-3-yle.

19. Composé selon la revendication 1, caractérisé en ce que R¹ représente un atome d'hydrogène, R² représente un groupe phtalimido, R³ représente un groupe H₂C=C(Me)-CH-COOCH₂C₆H₄NO₂-p, R⁴ représente un atome d'hydrogène et R⁵ représente un groupe 5-méthylisoxazol-3-yle.

20. Composé selon la revendication 1, caractérisé en ce que R¹ représente un atome d'hydrogène, R² représente un groupe phtalimido, R³ représente un groupe Me₂C=C-COOCH₂C₆H₄NO₂-p, R⁴ représente un atome d'hydrogène et R⁵ représente un groupe 5-méthyl-isoxazol-3-yle.

21. Composé selon la revendication 1, caractérisé en ce que R¹ représente un atome d'hydrogène, R² représente un groupe phényl-CH₂CONH, R³ représente un atome d'hydrogène, R⁴ représente un atome d'hydrogène et R⁵ représente un groupe phényl-CH₂.

22. Composé selon la revendication 1, caractérisé en ce que R¹ représente un atome d'hydrogène, R² représente un groupe phényl-CH₂CONH, R³ représente un groupe Me₂C=C-COOCH₂C₆H₄NO₂-p, R⁴ représente un atome d'hydrogène et R⁵ représente un groupe 5-méthyl-isoxazol-3-yle.

23. Composé selon la revendication 1, caractérisé en ce que R¹ représente un atome d'hydrogène, R² représente un groupe phényl-CH₂CONH, R³ représente un groupe Me₂C=C-COOH, R⁴ représente un atome d'hydrogène et R⁵ représente un groupe méthyl-isoxazol-3-yle.

24. Composé selon la revendication 1, caractérisé en ce que R¹ représente un atome d'hydrogène, R² représente un atome d'hydrogène, R³ représente un groupe Me₂C=C-COOH, R⁴ représente un atome d'hydrogène et R⁵ représente un groupe phényl-CH₂.

25. Composé selon la revendication 1, caractérisé en ce que R¹ représente un atome d'hydrogène, R² représente un groupe 3-o-chlorophényl-5-méthyl-isoxazol-4-yle, R³ représente un groupe H₂C=C(Me)-CH-COOCH₂C₆H₄Me-m, R⁴ représente un atome d'hydrogène et R⁵ représente un groupe 5-méthyl-isoxazol-3-yle.

26. Composé selon la revendication 1, caractérisé en ce que R¹ représente un atome d'hydrogène, R² représente un groupe 3-o-chlorophényl-5-méthyl-isoxazol-4-yle, R³ représente un groupe Me₂C=C-COOCH₂C₆H₄Me-m, R⁴ représente un atome d'hydrogène et R⁵ représente un groupe 5-méthyl-isoxazol-3-yle.

27. Composé selon la revendication 1, caractérisé en ce que R¹ représente un atome d'hydrogène, R² représente un groupe phtalimido, R³ représente un groupe Me₂C=C-COOH, R⁴ représente un atome d'hydrogène et R⁵ représente un groupe 5-méthyl-isoxazol-3-yle.

28. Composé selon la revendication 1, caractérisé en ce que R¹ représente un atome d'hydrogène, R² représente un groupe amino, R³ représente un groupe Me₂C=C-COOCH₂C₆H₄NO₂-p, R⁴ représente un atome d'hydrogène et R⁵ représente un groupe 5-méthyl-isoxazol-3-yle.

29. Composé selon la revendication 1, caractérisé en ce que R¹ représente un atome d'hydrogène, R² représente un groupe o-MeNHCOC₆H₄CONH, R³ représente un groupe Me₂C=C-COOCH₂C₆H₄NO₂-p, R⁴ représente un atome d'hydrogène et R⁵ représente un groupe méthyle.

30. Composé selon la revendication 1, caractérisé en ce que R¹ représente un atome d'hydrogène, R² représente un groupe o-MeNHCOC₆H₄CONH, R³ représente un groupe H₂C=C(Me)-CH-COO-CH₂C₆H₄NO₂-p, R⁴ représente un atome d'hydrogène et R⁵ représente un groupe méthyle.

31. Composé selon la revendication 1, caractérisé en ce que R¹ représente un atome d'hydrogène, R² représente un groupe o-MeNHCOC₆H₄CONH, R³ représente un groupe Me₂C=C-COOH, R⁴ représente un atome d'hydrogène et R⁵ représente un groupe méthyle.

32. Composé selon la revendication 1, caractérisé en ce que R¹ représente un atome d'hydrogène, R² représente un groupe phényl-CH₂CONH, R³ représente un groupe Me₂C=C-COOH, R⁴ représente un atome d'hydrogène et R⁵ représente un groupe phényl-CH₂.

33. Composé selon la revendication 1, caractérisé en ce que R¹ représente un atome d'hydrogène, R² représente un groupe phényl-CH₂CONH, R³ représente un groupe Me₂C=C-COOCH₂C₆H₄NO₂-p, R⁴ représente un atome d'hydrogène et R⁵ représente un groupe phényl-CH₂.

34. Composé selon la revendication 1, caractérisé en ce que R¹ représente un atome d'hydrogène, R² représente un groupe phényl-CH₂CONH, R³ représente un groupe Me₂C=C-COOCH₂C₆H₄NO₂-p, R⁴ représente un atome d'hydrogène et R⁵ représente un groupe méthyle.

35. Composé selon la revendication 1, caractérisé en ce que R¹ représente un atome d'hydrogène, R² représente un groupe phényl-CH₂CONH, R³ représente un groupe Me₂C=C-COOH, R⁴ représente un atome d'hydrogène et R⁵ représente un groupe méthyle.

36. Composé selon la revendication 1, caractérisé en ce que R¹ représente un atome d'hydrogène, R² représente un atome d'hydrogène, R³ représente un groupe Me₂C=C-COOH, R⁴ représente un atome d'hydrogène et R⁵ représente un groupe 5-méthyl-isoxazol-3-yle.

37. Composé selon la revendication 1, caractérisé en ce que R¹ représente un atome de brome, R² représente un atome de brome, R³ représente un groupe Me₂C=C-COOH, R⁴ représente un atome d'hydrogène et R⁵ représente un groupe phényl-CH₂.

38. Composé selon la revendication 1, caractérisé en ce que R¹ représente un atome d'hydrogène, R² représente un groupe phényl-OCH₂CONH, R³ représente un groupe Me₂C=C-COOH, R⁴ représente un atome d'hydrogène et R⁵ représente un groupe 5-méthyl-isoxazol-3-yle.

39. Procédé pour la préparation d'amides de l'acide 4-oxo-azétidin-2-sulfonique et de leurs sels répondant à la formule générale I dans laquelle les radicaux ont les significations indiquées à la revendication 1, caractérisé en ce qu'on oxyde des amides de l'acide 4-oxo-azétidin-2-sulfinique répondant à la formule générale II 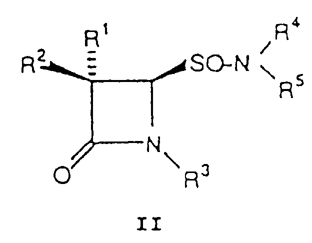 dans laquelle les radicaux ont les significations ci-après:
R¹ représente un atome d'hydrogène ou un atome d'halogène;
R² représente un atome d'hydrogène, un atome d'halogène, un groupe phényl-CH₂CONH, un groupe phényl-OCH₂CONH, un groupe phtalimido, un groupe o-MeNHCOC₆H₄CONH, un groupe isoxazolylcarbonylamino;
R³ représente un groupe Me₂C=C-COOOMe, un groupe H₂C=C-(Me)-CH-COOMe, un groupe Me₂C=C-COOCH₂phényle, un groupe H₂C=C(Me)-CH-COOCH₂C₆H₄NO₂-p, un groupe Me₂C=C-COOCH₂C₆H₄NO₂-p, un groupe Me₂C=C-COOCH₂-C₆H₄Me-m, un groupe H₂C=C(Me)-CH-COOCH₂C₆H₄Me-m;
R⁴ représente un atome d'hydrogène; et
R⁵ représente un groupe alkyle, un groupe benzyle, un groupe isoxazole ou un groupe pyrazole,
dans les agents habituels pour les oxydations connues dans la chimie organique, tels que par exemple le peroxyde d'hydrogène, l'acide peracétique, l'acide m-chlorobenzoïque et le permanganate de potassium, dans un milieu acide ou neutre, aqueux ou aqueux-organique et à une température de 0 à 100°C, puis on procède de la manière habituelle à l'élimination des groupes de protection, au traitement du mélange réactionnel et à l'isolation du produit.

40. Utilisation du composé selon la revendication 1 à titre de composé intermédiaire pour la synthèse d'analogues de bêta-lactames, en particulier de systèmes bicycliques.

41. Utilisation du composé selon la revendication 1 à titre de principe actif pour la formulation de préparations destinées à la thérapie antimicrobienne.
